# EUROPEAN PATENT SPECIFICATION

(11) **EP 0 669 978 B1**
(45) Date of publication and mention of the grant of the patent: **23.04.2003**
(21) Application number: 94900810.6
(22) Date of filing: 15.11.1993
(51) Int. Cl.: C12N 15/12, C07K 14/00, C12P 21/08, C12N 15/86, C12Q 1/68, G01N 33/567, A61K 38/00, C12N 15/62

(54) **FLK-1 IS A RECEPTOR FOR VASCULAR ENDOTHELIAL GROWTH FACTOR**
FLK-1 IST EIN REZEPTOR FÜR DEN WACHSTUMSFAKTOR DER VASKULAREN ENDOTHELZELLEN
Flk-1 UTILISE COMME RECEPTEUR POUR FACTEUR DE CROISSANCE ENDOTHELIAL VASCULAIRE

(30) Priority: 13.11.1992 US 975750; 26.03.1993 US 38596
(43) Date of publication of application: 06.09.1995
(73) Proprietor: Max-Planck-Gesellschaft zur Förderung der Wissenschaften e.V., D-37073 Göttingen (DE)
(72) Inventor: ULLRICH, Axel, Portola Valley, California 94028 (US); RISAU, Werner, D-35510 Butzbach (DE); MILLAUER, Birgit, D-81241 München (DE)
(74) Representative: Patentanwälte Ruff, Wilhelm, Beier, Dauster & Partner
(86) International application number: EP9303191
(87) International publication number: WO94011499

(56) References cited:
- WO-A-92/03459
- WO-A-92/17486
- PROCEEDINGS OF THE NATIONAL ACADEMY OF SCIENCES OF USA. vol. 88 , October 1991 , WASHINGTON US pages 9026 - 9030 MATTHEWS, W. ET AL.; 'A receptor tyrosine kinase cDNA isolated from a population of enriched primitive hematopoietic cells and exhibiting close genetic linkage to c-kit.'
- BIOTECHNOLOGY vol. 3, no. 8 , August 1985 , NEW YORK US pages 689 - 693 MC CORMICK, D.; 'Human gene therapy : the first round'
- CELL vol. 72 , 26 March 1993 , CAMBRIDGE, NA US pages 835 - 846 MILLAUER, B., WIZIGMANN-VOOS, S., SCHNURCH, H., MARTINEZ, R., MOLLER, N.P., RISAU, W., AND ULLRICH, A.; 'High affinity VEGF binding and developmental expression suggest Flk-1 as a major regulator of vasculogenesis and angiogenesis.'

## Description

### 1. INTRODUCTION

The present invention relates to the use of a recombinant vector comprising a nucleotide sequence encoding a Flk-1 receptor having a mutation in the cytoplasmic kinase domain for the modulation of angiogenesis and vasculogenesis. The invention is based, in part, an the demonstration that Flk-1 tyrosine kinase receptor expression is associated with endothelial cells and the identification of vascular endothelial growth factor (VEGF) as the high affinity ligand of Flk-1. These results indicate a major role for Flk-1 in the signaling system during vasculogenesis and angiogenesis. Engineering of host cells that express Flk-1 and the uses of expressed Flk-1 to evaluate and screen for drugs and analogs of VEGF involved in Flk-1 modulation by either agonist or antagonist activities is described.

### 2. BACKGROUND OF THE INVENTION

Receptor tyrosine kinases comprise a large family of transmembrane receptors for polypeptide growth factors with diverse biological activities. Their intrinsic tyrosine kinase function is activated upon ligand binding, which results in phosphorylation of the receptor and multiple cellular substrates, and subsequently in a variety of cellular responses (Ullrich A. and Schlessinger, J., 1990, Cell 61:203-212).

A receptor tyrosine kinase cDNA designated fetal liver kinase 1 (Flk-1), was cloned from mouse cell populations enriched for hematopoietic stem and progenitor cells. The receptor was suggested to be involved in hematopoietic stem cell renewal (Matthews et al., 1991, Proc. Natl. Acad. Sci. USA 88:9026-9030). Sequence analysis of the Flk-1 clone revealed considerable homology with the c-Kit subfamily of receptor kinases and in particular to the *Flt* gene product. These receptors all have in common an extracellular domain containing immunoglobulin-like structures.

The formation and spreading of blood vessels, or vasculogenesis and angiogenesis, respectively, play important roles in a variety of physiological processes such as embryonic development, wound healing, organ regeneration and female reproductive processes such as follicle development in the corpus luteum during ovulation and placental growth after pregnancy. Uncontrolled angiogenesis can be pathological such as in the growth of solid tumors that rely on vascularization for growth.

Angiogenesis involves the proliferation, migration and infiltration of vascular endothelial cells, and is likely to be regulated by polypeptide growth factors. Several polypeptides with in vitro endothelial cell growth promoting activity have been identified. Examples include acidic and basic fibroblastic growth factor, vascular endothelial growth factor and placental growth factor. Although four distinct receptors for the different members of the FGF family have been characterized, none of these have as yet been reported to be expressed in blood vessels in vivo.

While the FGFs appear to be mitogens for a large number of different cell types, VEGF has recently been reported to be an endothelial cell specific mitogen (Ferrara, N. and Henzel, W.J.,1989, Biochem. Biophys. Res. Comm. 161:851-858). Recently, the fms-like tyrosine receptor, *flt*, was shown to have affinity for VEGF (DeVries, C. et al. ,1992, Science 255:989-991).

### 3. SUMMARY OF THE INVENTION

The present invention relates to the use of a recombinant vector comprising a nucleotide sequence encoding a flk-1 receptor having a mutation in the cytoplasmic kinase domain for the modulation of angiogenesis and vasculogenesis. The present invention is based, in part, on the discovery that the Flk-1 tyrosine kinase receptor is expressed on the surface of endothelial cells and the identification of vascular endothelial growth factor (VEGF) as the high affinity ligand of Flk-1. The role of endothelial cell proliferation and migration during angiogenesis and vasculogenesis indicate an important role for Flk-1 in these processes. The invention is described by way of example for the murine Flk-1, however, the principles may be applied to other species including humans.

The invention relates to expression systems designed to produce Flk-1 protein and/or cell lines which express the Flk-1 receptor according to the invention. Expression of soluble recombinant Flk-1 protein may be used to screen peptide libraries for molecules that inhibit the Flk-1/VEGF interaction. Engineered cell lines expressing Flk-1 on their surface may be advantageously used to screen and identify VEGF agonists and antagonists.

### 4. BRIEF DESCRIPTION OF THE FIGURES

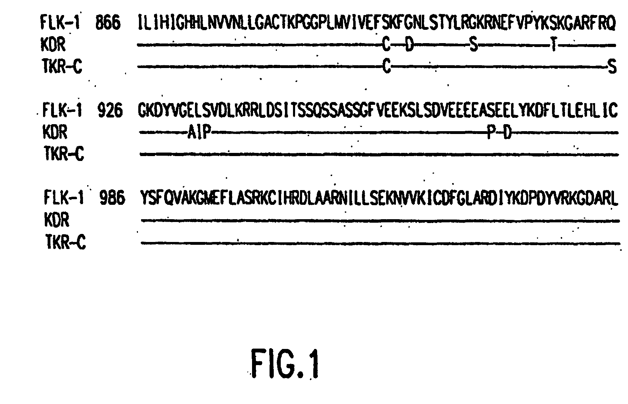

FIG. 1. Comparison of the Flk-1 amino acid sequence with related RTKs. Amino acid sequence comparison of Flk-1 with human KDR and rat TKr-C. A section of the sequence which is known for all three receptors is compared and only differences to the Flk-1 sequence are shown.

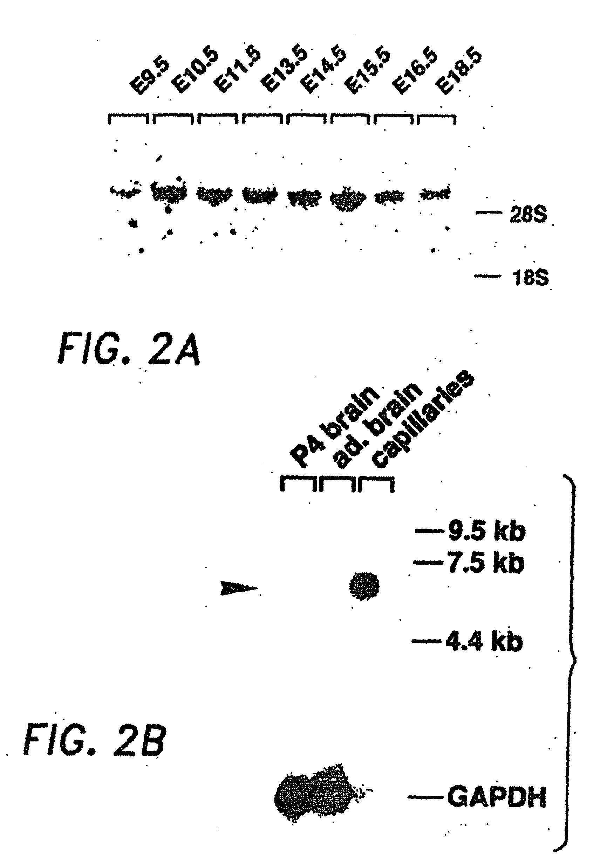

FIG. 2. Northern blot analysis of Flk-1 gene expression. (A) Expression of Flk-1 RNA in day 9.5 to day 18.5 mouse embryos. Samples (10 µg) of total RNA from whole mouse embryos were analyzed in each lane. Positions of 28S and 18S ribosomal RNAs are marked. (B) Expression of Flk-1 mRNA in postnatal day 4 and adult brain in comparison with capillary fragments from postnatal day 4 brain. 1µg of poly (A⁺) RNA was loaded on each lane. The 5' 2619 bp of the Flk-1 cDNA were used as a probe. Control hybridization with a GAPDH cDNA probe is shown in the lower panel.

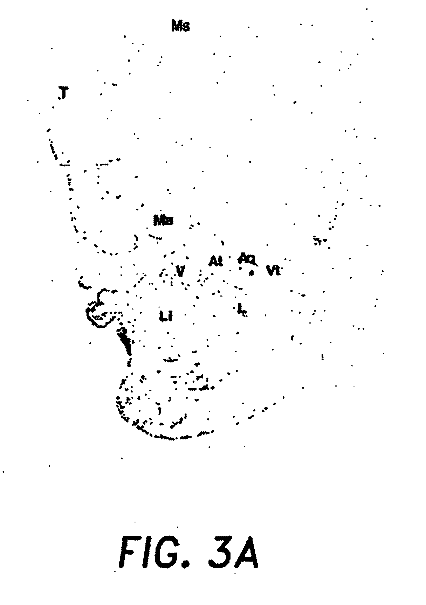

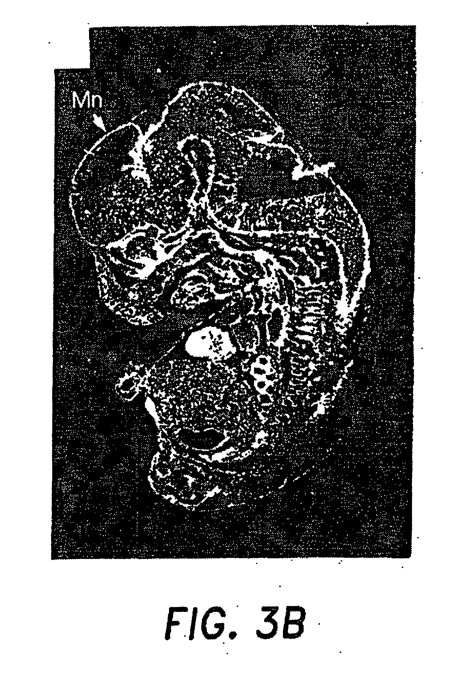

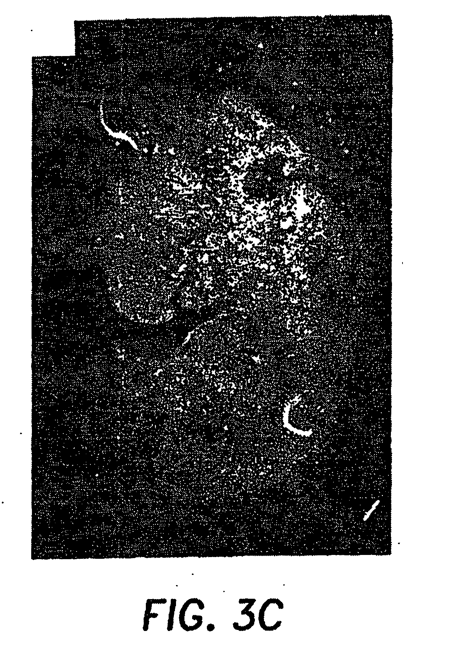

FIG. 3. Abundant Flk-1 gene expression in embryonic tissues. *In situ* hybridization analysis of Flk-1 expression in day 14.5 mouse embryo. (A) Bright field illumination of a parasagittal section through the whole embryo hybridized with a ³⁵S-labeled antisense probe (5' 2619 bp). (B) Dark field illumination of the same section. (C) Control hybridization of an adjacent section with a sense probe. Abbreviations: Ao, aorta; At, atrium; L, lung; Li, liver; Ma, mandible; Mn, meninges; Ms. mesencephalon; T, telencephalon; V, ventricle; Vt, vertebrae.

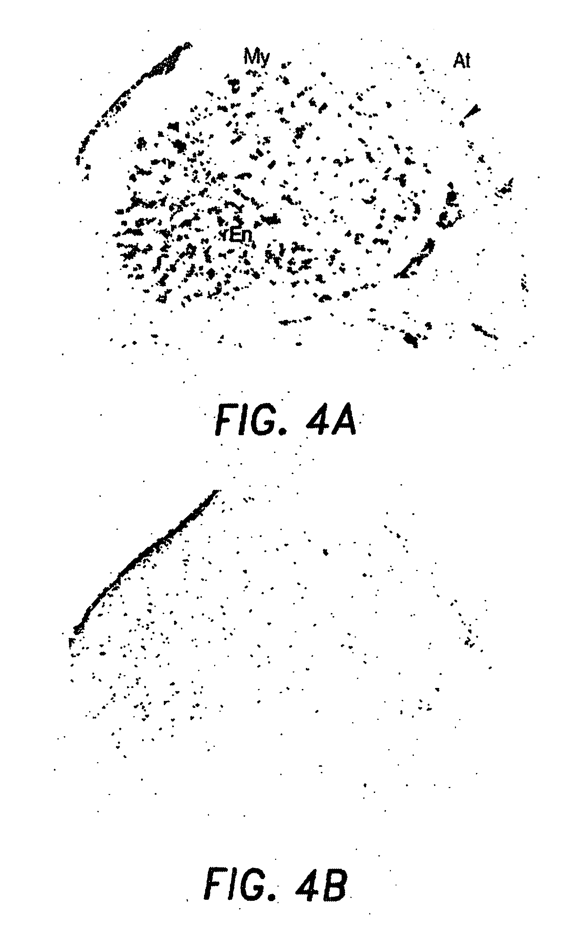

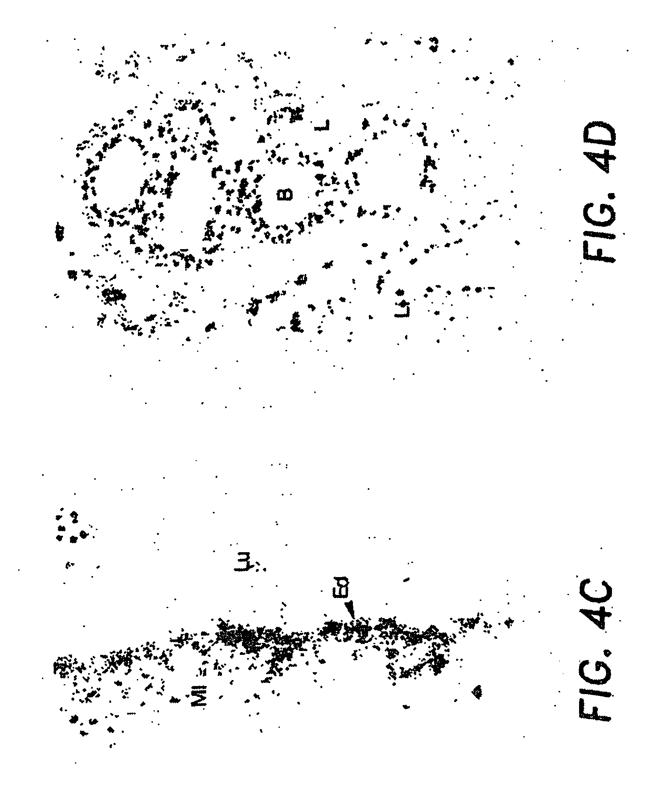

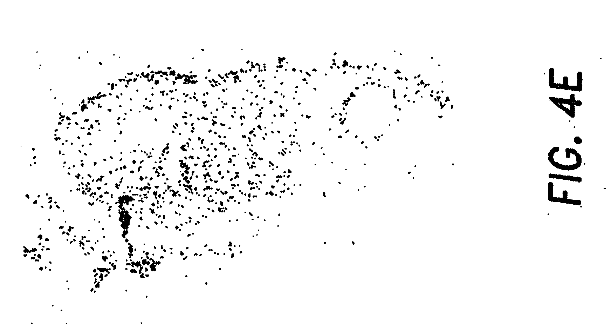

FIG. 4. Expression of Flk-1 RNA in embryonic organs is restricted to specific cells. Expression of Flk-1 RNA in a day 14.5 mouse embryo at higher magnification. (A) The heart region was probed with a ³⁵S-labeled antisense probe. (B) Adjacent section hybridized with the sense probe. (C) Part of the aorta wall shown on the cellular level. The endothelial cell-layer is indicated by an arrow. (D) The lung, probed with the Flk-1 antisense probe. (E) Control hybridization of an adjacent section hybridized with the sense probe. Abbreviations: At, atrium; B, bronchus; Ed, endothelial cell layer; En, endocardium; L, lung, Li, liver; Lu, lumina of the aorta; Ml, muscular; My, myocardium.

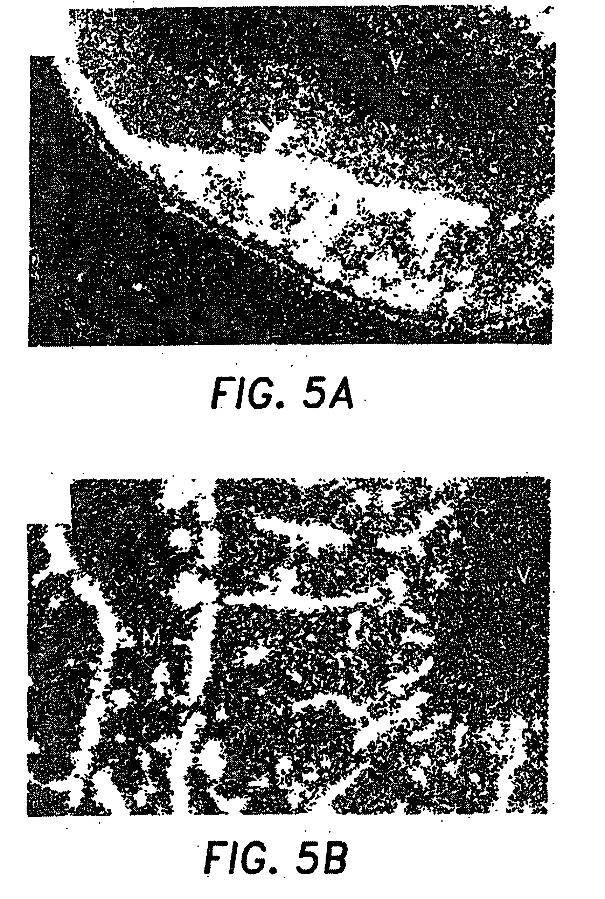

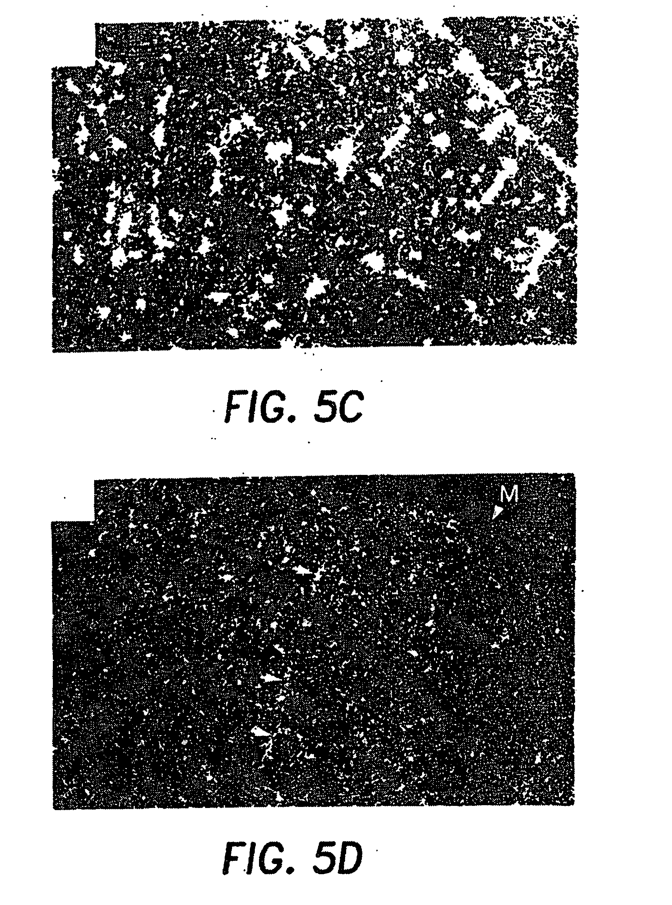

FIG. 5. Flk-1 gene expression in the brain of the developing mouse. *In situ* hybridization analysis of Flk-1 gene expression in the brain at different developmental stages. All sections were probed with the Flk-1 antisense probe. (A) Sagittal section of the telencephalon of a day 11.5 mouse embryo. A single blood vessel expressing Flk-1, which sprouts from the meninges into the neuroectoderm, is indicated by an arrow. (B) Sagittal sections of the brain of embryo day 14.5 and (C) of postnatal day 4. Shown are regions of the mesencephalon. Branching capillaries and blood vessels expressing Flk-1 are indicated by an arrow. (D) Sagittal section of an adult brain; a region of the mesencephalon is shown. Cells expressing Flk-1 are indicated by an arrow. Abbreviations: M, meninges; V, ventricle;

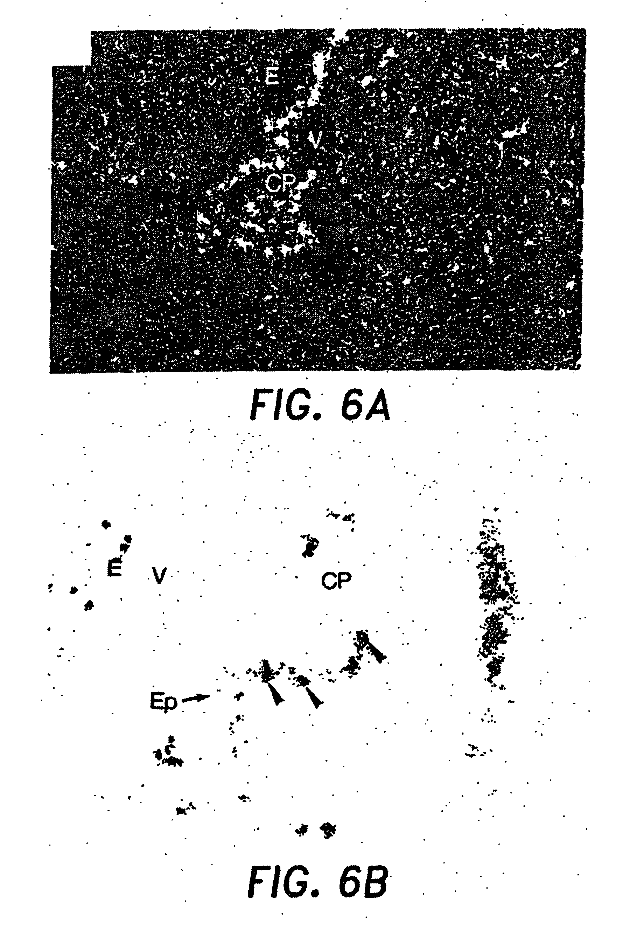

FIG. 6. Expression of Flk-1 in the choroid plexus of adult brain. (A) Darkfield illumination of the choroid plexus of an adult mouse brain hybridized with Flk-1 antisense probe. (B) Choroid plexus shown at a higher magnification. Arrows indicate single cells, which show strong expression of Flk-1. Abbreviations: CP, choroid plexus; E, ependyme; Ep, epithelial cells; V, ventricle.

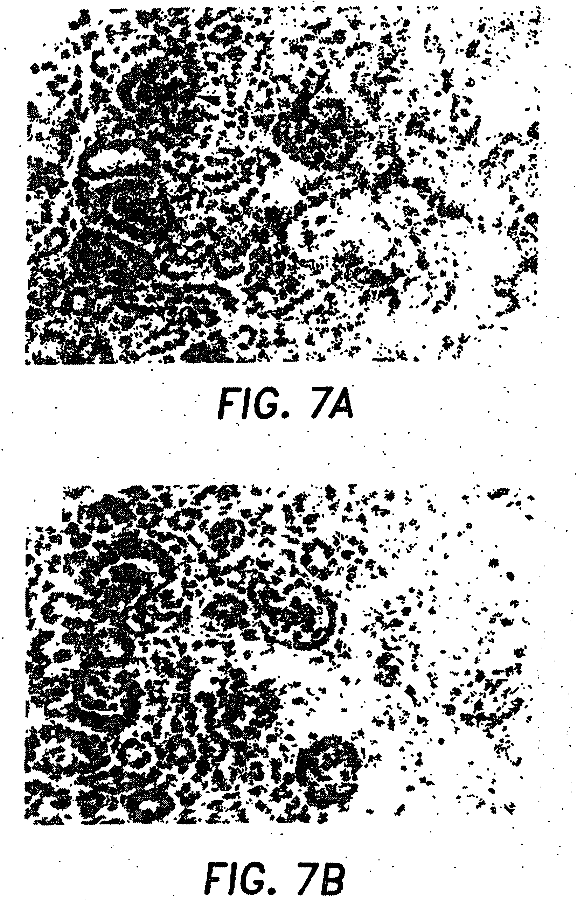

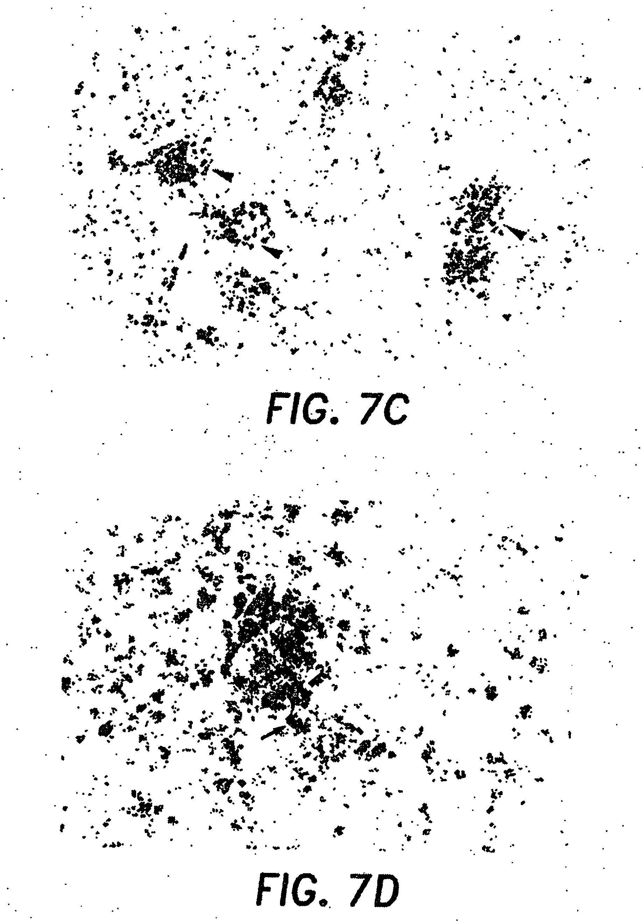

FIG. 7. Flk-1 is expressed in the glomeruli of the kidney. (A) Parasagittal section of a 4-day postnatal kidney, hybridized with the Flk-1 antisense probe. Hybridization signal accumulates in the glomeruli, as . indicated by arrowheads. (B) Control hybridization of an adjacent section with the sense probe. (C) Sagittal section of an adult kidney probed with Flk-1. Arrowheads indicate glomeruli. (D) Glomerulus of an adult kidney at a higher magnification. The arrows in (A) and (D) indicate cells aligned in strands in the juxtaglomerular region expressing Flk-1.

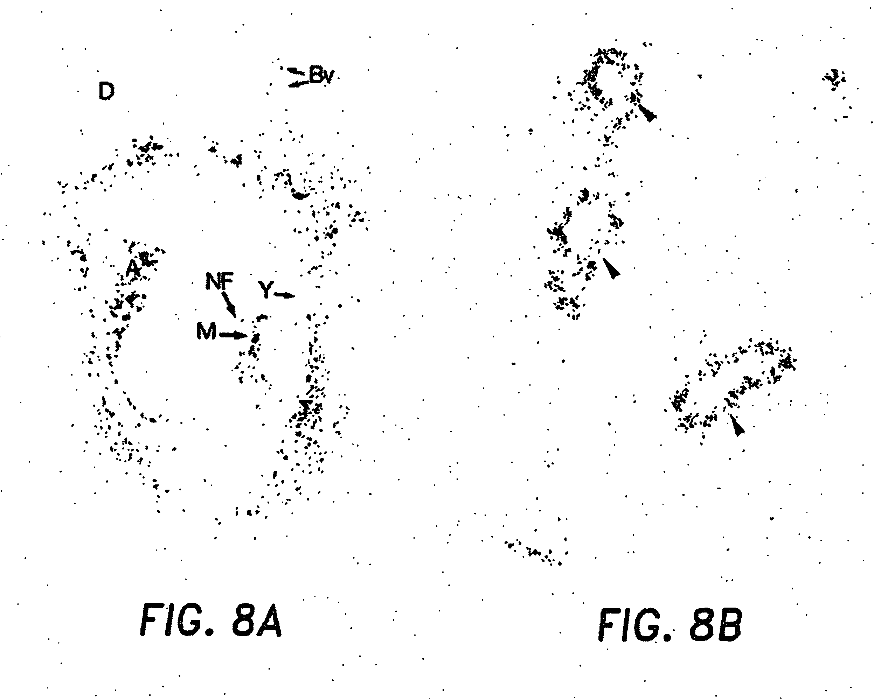

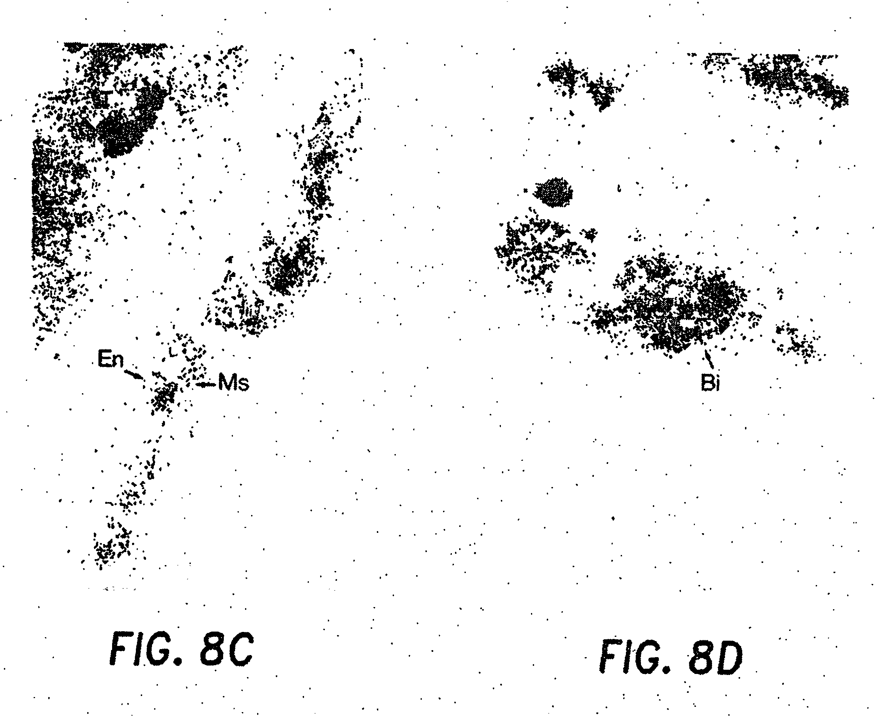

FIG. 8. *In situ* hybridization analysis of Flk-1 expression in early embryos and extraembryonic tissues. (A) Sagittal section of a day 8.5 mouse embryo in the maternal deciduum probed with Flk-1. (B) Higher magnification of the deciduum. Arrowheads indicate the endothelium of maternal blood vessels strongly expressing Flk-1 RNA. (C) High magnification of the yolk sac and the trophectoderm of a day 9.5 mouse embryo. (D) High magnification of a blood island. Abbreviations: A, allantois; Bi, blood island; Bv, maternal blood vessel; D, deciduum; En, endodermal layer of yolk sac; M, mesenchyme; Ms, mesodermal layer of yolk sac; NF, neural fold; T, trophoblast; Y, yolk sac.

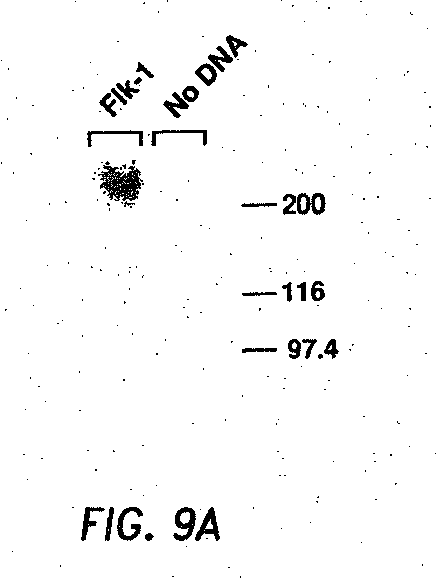

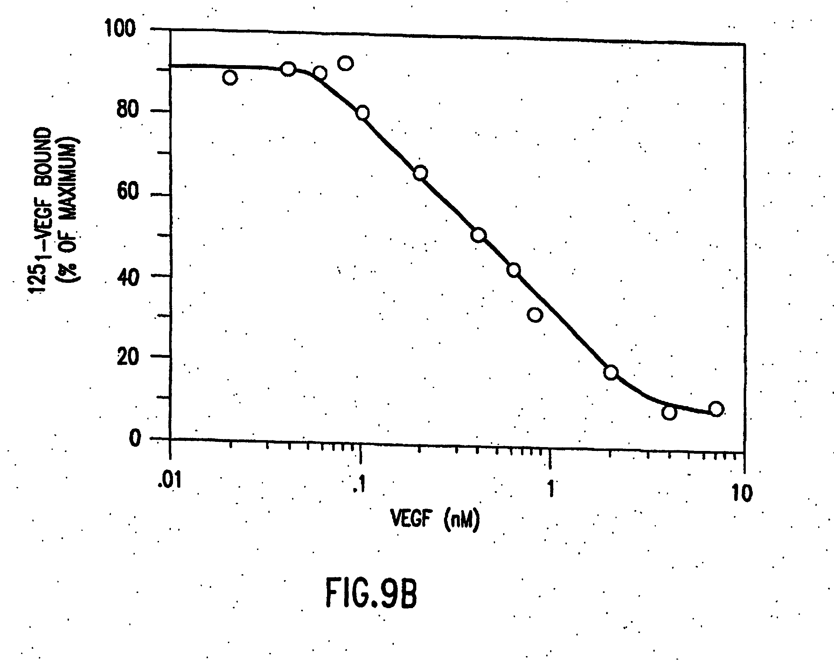

FIG. 9. Flk-1 is a receptor for VEGF. (A) Cross linking of ¹²⁵I-VEGF to COS cells transiently expressing the Flk-1 receptor and control cells were incubated with ¹²⁵I-VEGF at 4°C overnight, then washed twice with phosphate buffered saline (PBS) and exposed to 0.5 mM of the cross linking agent DSS in PBS for 1 hour at 4°C. The cells were lysed, Flk-1 receptor immunoprecipitated, and analyzed by polyacrylamide gel electrophoresis followed by autoradiography. Molecular size markers are indicated in kilodaltons. (B) Specific binding of ¹²⁵I-VEGF to COS cells expressing Flk-1. COS cells transiently expressing Flk-1 were removed from the plate and resuspended in binding medium (DMEM, 25 mM Hepes, 0.15% gelatin). Binding was performed at 15°C for 90 minutes in a total volume of 0.5 ml containing 2x10⁵ cells, 15,000 cpm ¹²⁵I-VEGF, and the indicated concentrations of unlabeled ligand. The cells were washed twice with PBS / 0.1% BSA and counted in a gamma counter.

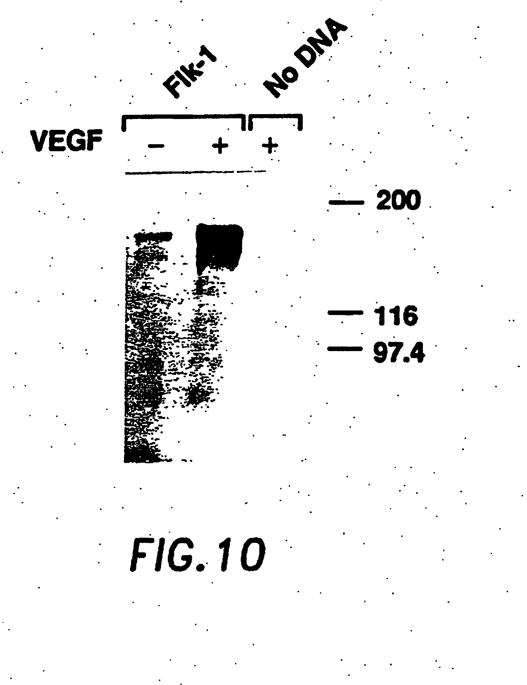

FIG. 10. VEGF-induced autophosphorylation of Flk-1. COS cells transiently expressing Flk-1 receptor and control cells were starved for 24 hours in DMEM containing 0.5% fetal calf serum and then stimulated with VEGF for 10 minutes as indicated. The cells were solubilized, Flk-1 receptor immunoprecipitated with a polyclonal antibody against its C-terminus, separated by polyacrylamide gel electrophoresis, and transferred to nitrocellulose. The blot was probed with antiphosphotyrosine antibodies (5B2). The protein bands were visualized by using a horseradish-peroxidase coupled secondary antibody and BCL™ (Amersham) detection assay.

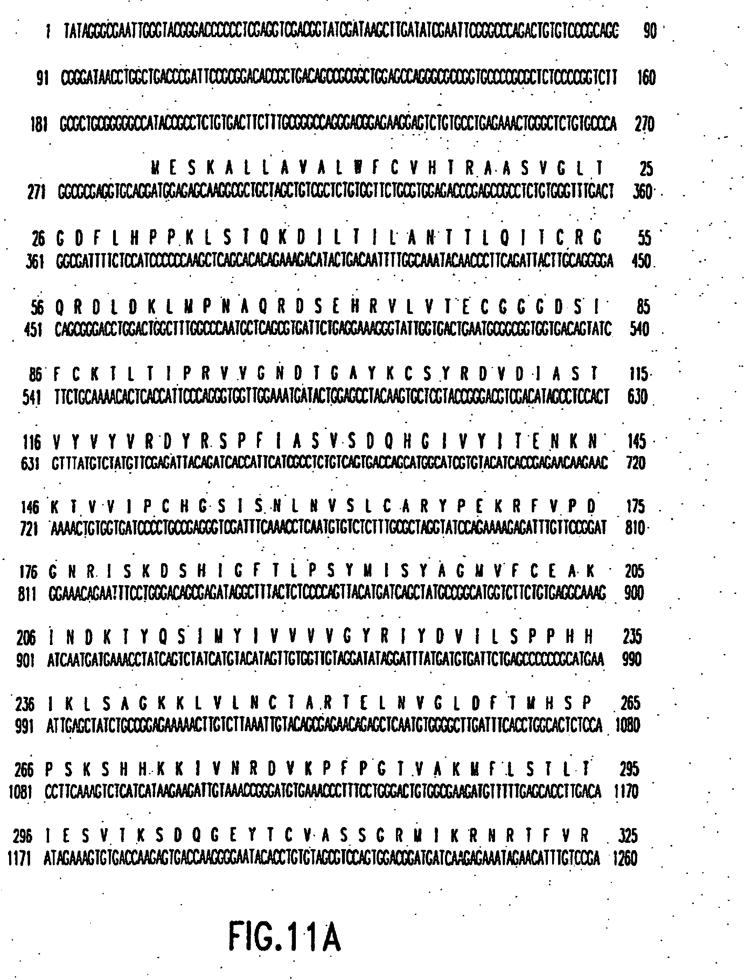

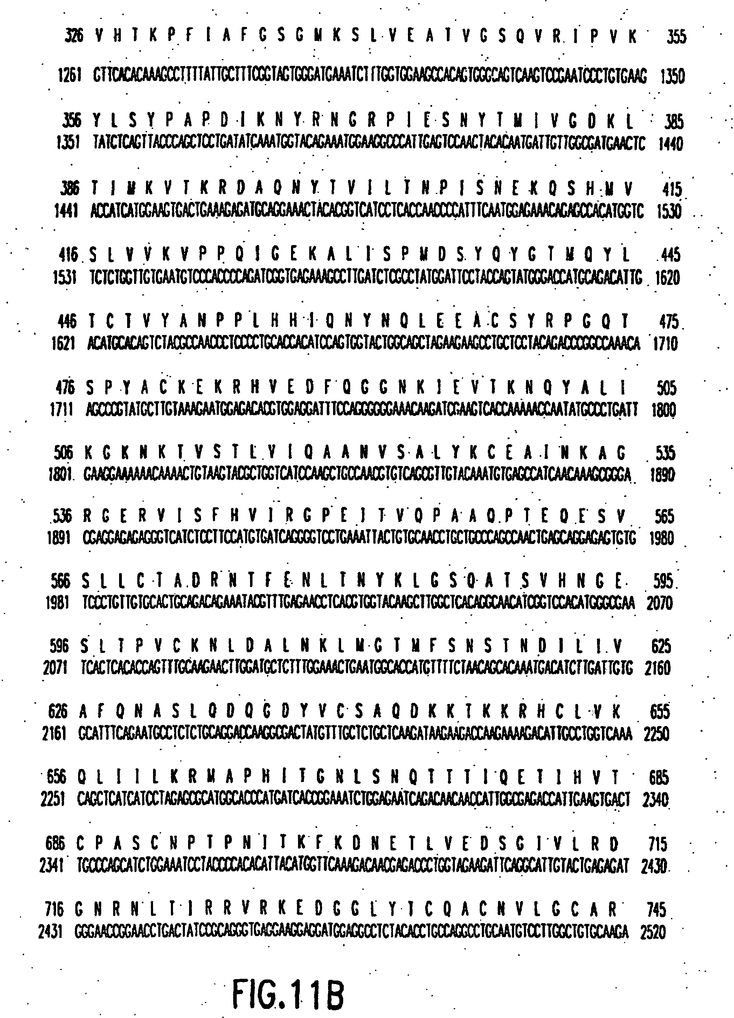

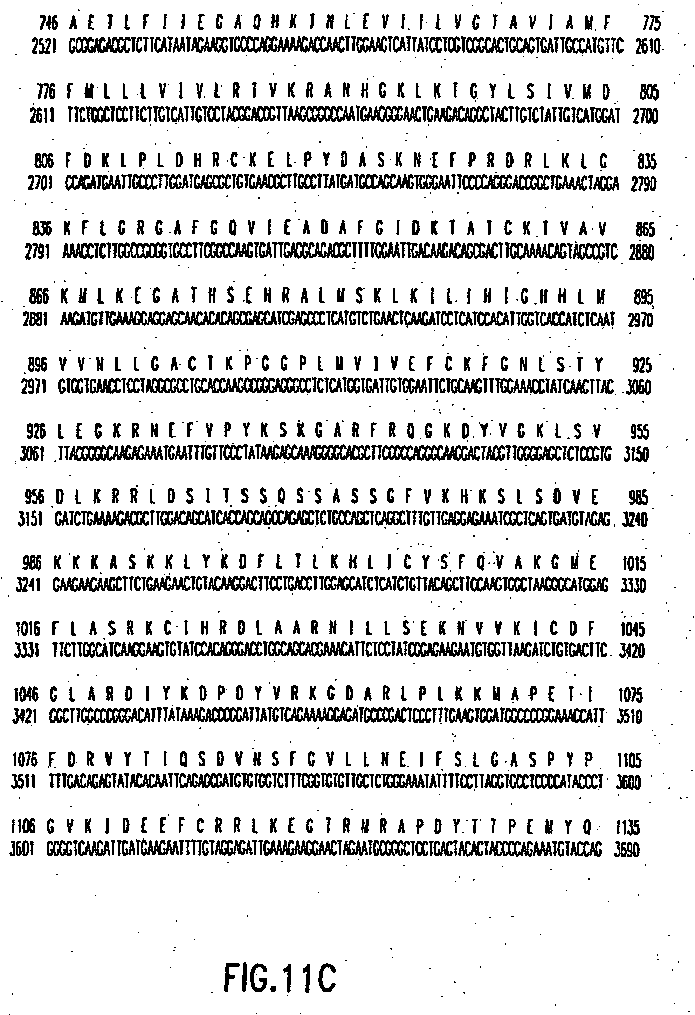

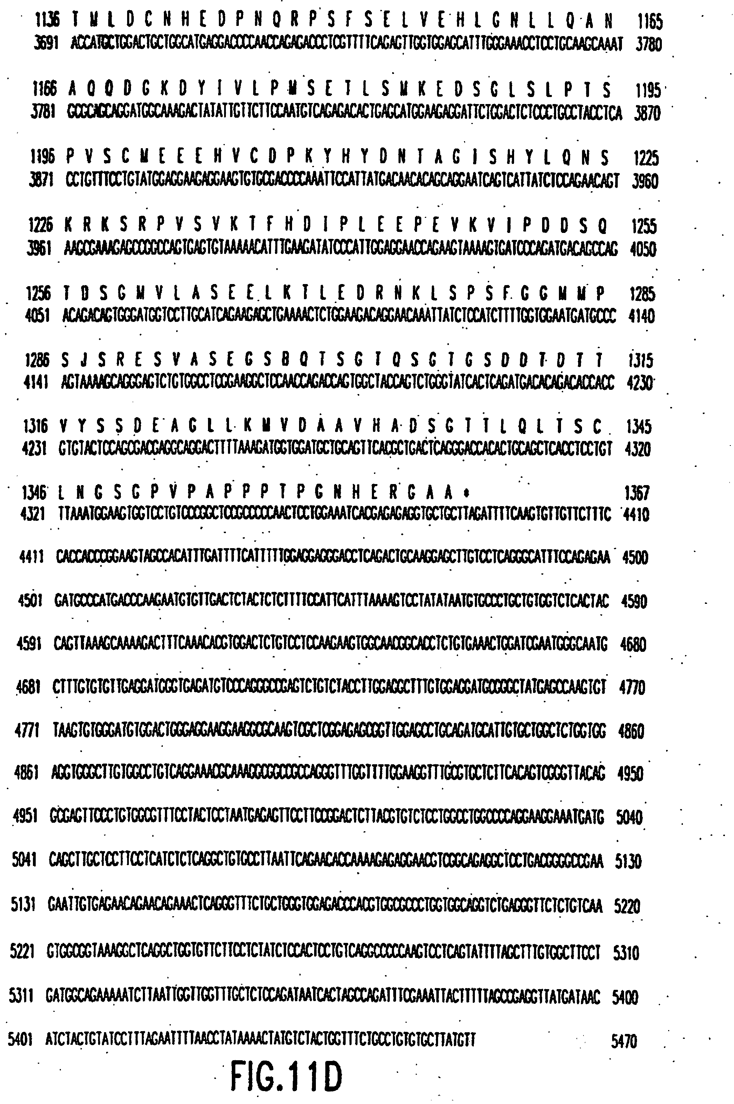

FIG. 11. Nucleotide Sequence of Murine Flk-1.

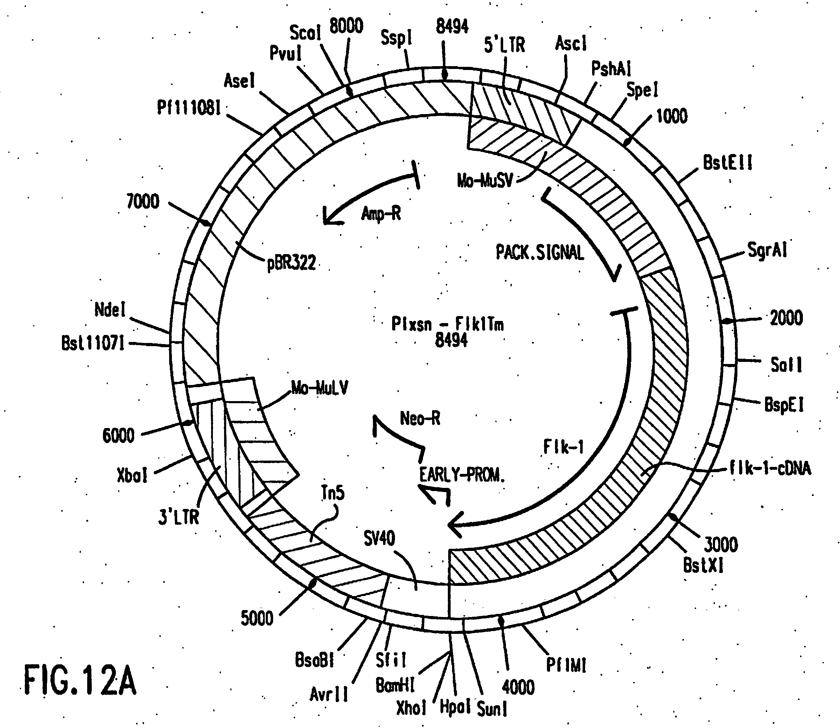

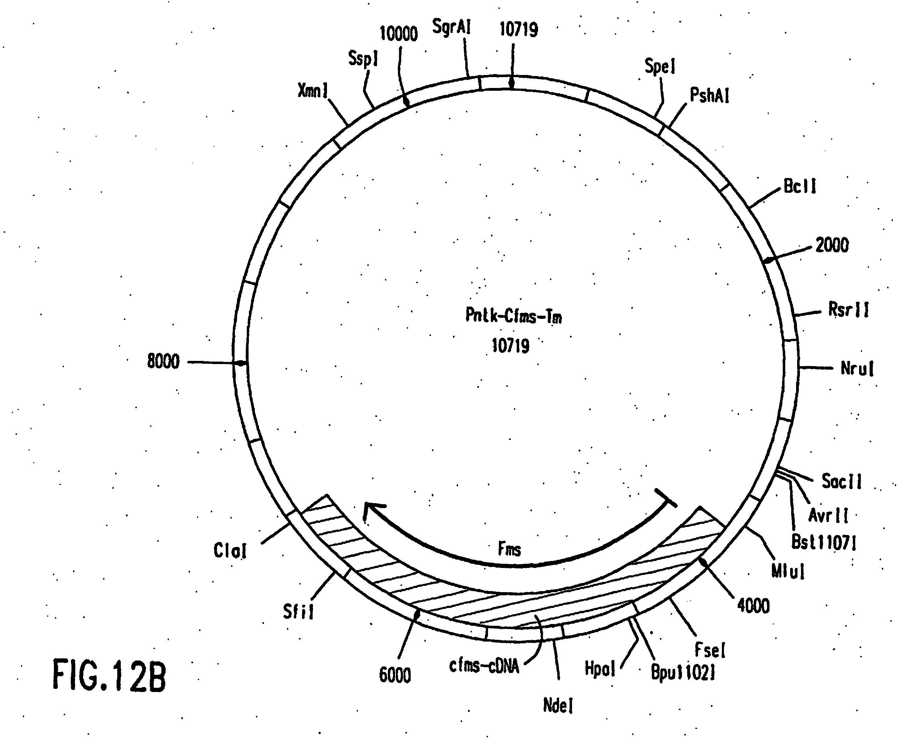

FIG. 12. Plasmid Maps of retroviral vector constructs. pLXSN Flk-1 TM Cl.1 and pLXSN Flk-1 TM cl.3 contain Flk-1 amino acids 1 through 806. pNTK-cfms-TM contains the 541 N-terminal amino acids of c-fms.

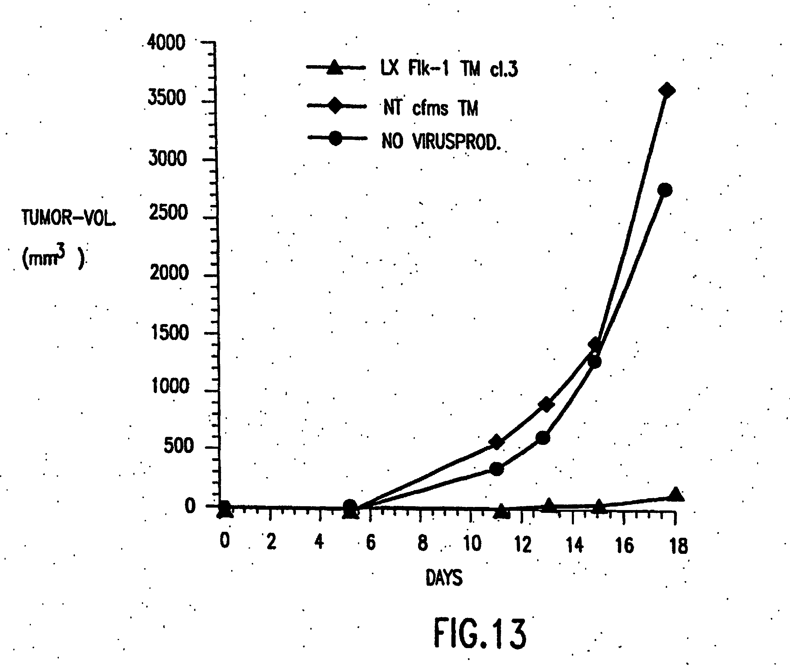

FIG. 13. Inhibition of C6 glioblastoma tumor growth by transdominant-negative inhibition of Flk-1. C6 cells were implanted either alone or coimplanted with virus-producing cells. Cell numbers are as indicated in each panel. Two different virus-producing cells lines were used: one expressing the Flk-1 TM (transdominant-negative) mutant and one expressing a transdominant-negative c-fms mutant (c-fms TM) as a control. Beginning at the time when the first tumors appeared, tumor volumes were measured every 2 to 3 days to obtain a growth curve. Each group is represented by four mice.

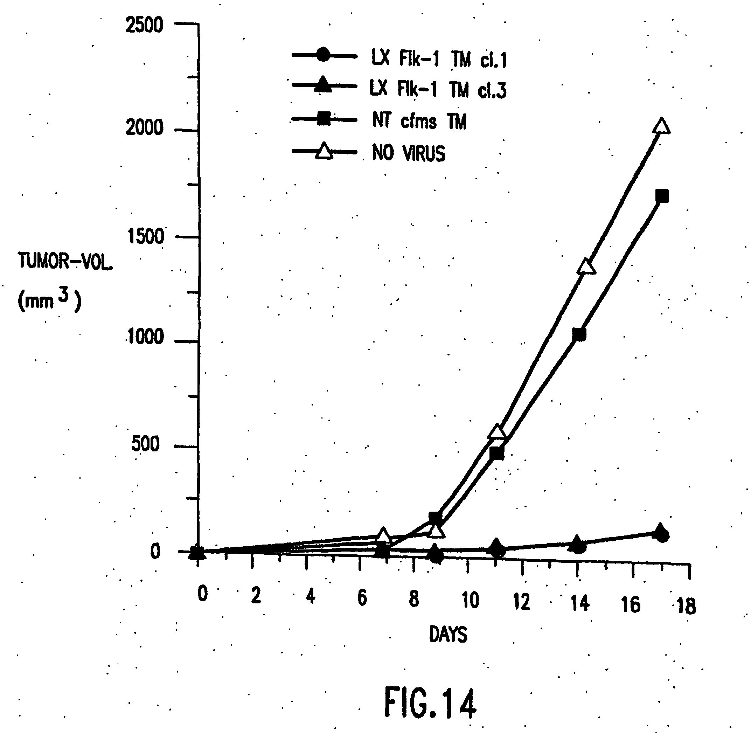

FIG. 14. Inhibition of C6 glioblastoma tumor growth by transdominant-negative inhibition of Flk-1. C6 cells were implanted either alone or coimplanted with virus-producing cells. Cell numbers are as indicated in each panel. Two different virus-producing cell lines were used: one expressing the Flk-1 TM (transdominant-negative) mutant and one expressing a transdominant-negative c-fms mutant (cfms TM) as a control. Beginning at the time when the first tumor appeared, tumor volumes were measured every 2 to 3 days to obtain growth curve. Each group is represented by four mice.

### 5. DETAILED DESCRIPTION OF THE INVENTION

The present invention relates to the use of recombinant vectors containing a nucleotide sequence encoding a flk-1 receptor having a mutation in the cytoplasmatic kinase domain to modulate angiogenesis and/or vasculogenesis.

The invention, is based, in part, on results from *in situ*-hybridization and Northern blot analyses indicating that Flk-1 is an endothelial cell specific RTK. In addition, cross-linking experiments have shown Flk-1 to be a high affinity receptor for vascular endothelial growth factor (VEGF), indicating that Flk-1 plays a crucial role in the development and differentiation of hemangioblast and in subsequent endothelial cell growth during vasculogenesis and angiogenesis.

The invention is based, on the discovery that expression of a transdominant-negative mutant form of the Flk-1 molecule can inhibit the biological activity of the endogenous wild type Flk-1. Experiments are descirbed herein, in which tumor cells and cells producing a recombinant retrovirus encoding a truncated Flk-1 receptor were mixed and injected into mice. Inhibition of vasculogenesis and growth of the injected tumor cells was observed in mice expressing the trucated form of the Flk-1 receptor. Expression of transdominant negative forms of the Flk-1 molecule may be useful for treatment of diseases resulting from abnormal proliferation of blood vessels, such as rheumatoid arthritis, retinopathies and growth of solid tumors.

As explained in the working examples, infra, the polymerase chain reaction (PCR) method was used to isolate new receptor tyrosine kinases specifically expressed in post-implantation embryos and endothelial cells. One such clone was found to encode a RTK that had almost identical sequence homology with the previously identified cDNA clone isolated from populations of cells enriched for hematopoietic cells and designated fetal liver kinase-1 (Flk-1) (Matthews et al., 1991, Proc. Natl. Acad Sci. U.S.A. 88:9026-9030) (FIG. 11).

For clarity of discussion, the invention is described in the subsections below by way of example for the murine Flk-1. However, the principles may be analogously applied to clone and express the Flk-1 of other species including humans.

### 5.1. THE Flk-1 CODING SEQUENCE

The nucleotide coding sequence and deduced amino acid sequence of the murine Flk-1 gene is depicted in Figure 11 (SEQ. ID NO. 1) and has recently been described in Matthews et al., 1991, Proc. Natl. Acad. Sci. U.S.A., 88:9026-9030. In accordance with the invention, the nucleotide sequence of the Flk-1 protein or its functional equivalent in mammals, including humans, can be used to generate recombinant molecules which direct the expression of Flk-1; hereinafter, this receptor will be referred to as "Flk-1", regardless of the species from which it is derived.

In a specific embodiment described herein, the murine Flk-1 gene was isolated by performing a polymerase chain reaction (PCR) using two degenerate oligonucleotide primer pools that were designed on the basis of highly conserved sequences within the kinase domain of receptor tyrosine kinases (Hanks et al., 1988,) As a template, DNA from a λgt10 cDNA library prepared from day 8.5 mouse embryos, was used. In a parallel approach, similar primers were used to amplify RTK cDNA sequences from capillary endothelial cells that had been isolated from the brains of post-natal day 4-8 mice. This is a time when brain endothelial cell proliferation is maximal. Both approaches yielded cDNA sequences encoding the recently described fetal liver RTK, Flk-1 (Matthews et al., 1991). Based on amino acid homology, this receptor is a member of the type III subclass of RTKs (Ullrich and Schlessinger) which contain immunoglobulin-like repeats in their extracellular domains (FIG. 1).

The invention also relates to Flk-1 genes isolated from other species, including humans, in which Flk-1 activity exists. Members of the Flk-1 family are defined herein as those receptors that bind VEGF or fragments of the peptide. Such receptors may demonstrate about 80% homology at the amino acid level in substantial stretches of DNA sequence. A bacteriophage cDNA library may be screened, under conditions of reduced stringency, using a radioactively labeled fragment of the mouse Flk-1 clone. Alternatively the mouse Flk-1 sequence can be used to design degenerate or fully degenerate oligonucleotide probes which can be used as PCR probes or to screen bacteriophage cDNA libraries. A polymerase chain reaction (PCR) based strategy may be used to clone human Flk-1. Two pools of degenerate oligonucleotides, corresponding to a conserved motifs between the mouse Flk-1 and receptor tyrosine kinases, may. be designed to serve as primers in a PCR reaction. The template for the reaction is cDNA obtained by reverse transcription of mRNA prepared from cell lines or tissue known to express human Flk-1. The PCR product may be subcloned and sequenced to insure that the amplified sequences represent the Flk-1 sequences. The PCR fragment may be used to isolate a full length Flk-1 cDNA clone by radioactively labeling the amplified fragment and screening a bacteriophage cDNA library. Alternatively, the labeled fragment may be used to screen a genomic library. For a review of cloning strategies which may be used, see e.g., Maniatis, 1989, Molecular Cloning, A Laboratory Manual, Cold Springs Harbor Press, N.Y.; and Ausubel et al., 1989, Current Protocols in Molecular Biology, (Green Publishing Associates and Wiley Interscience, N.Y.)

Isolation of a human Flk-1 cDNA may also be achieved by construction of a cDNA library in a mammalian expression vector such as pcDNA1, that contains SV40 origin of replication sequences which permit high copy number expression of plasmids when transferred into COS cells. The expression of Flk-1 on the surface of transfected COS cells may be detected in a number of ways, including the use of a labeled ligand such as VEGF or a VEGF agonist labeled with a radiolabel, fluorescent label or an enzyme. Cells expressing the human Flk-1 may be enriched by subjecting transfected cells to a FACS (fluorescent activated cell sorter) sort.

In accordance with the invention, Flk-1 nucleotide sequences which encode Flk-1, peptide fragments of Flk-1, Flk-1 fusion proteins or functional equivalents thereof may be used to generate recombinant DNA molecules according to the invention in appropriate host cells. Alternatively, nucleotide sequences which hybridize to portions of the Flk-1 sequence may also be used in nucleic acid hybridization assays, Southern and Northern blot analyses, etc.

Due to the inherent degeneracy of the genetic code, other DNA sequences which encode substantially the same or a functionally equivalent amino acid sequence, may be used in the practice of the invention for the cloning and expression of the Flk-1 protein. Such DNA sequences include those which are capable of hybridizing to the murine Flk-1 sequence under stringent conditions.

Altered DNA sequences which may be used in accordance with the invention include deletions, additions or substitutions of different nucleotide residues resulting in a sequence that encodes the same or a functionally equivalent gene product. The gene product itself may contain deletions, additions or substitutions of amino acid residues within the Flk-1 sequence, which result in a silent change thus producing a functionally equivalent Flk-1. Such amino acid substitutions may be made on the basis of similarity in polarity, charge, solubility, hydrophobicity, hydrophilicity, and/or the amphipatic nature of the residues involved. For example, negatively charged amino acids include aspartic acid and glutamic acid; positively charged amino acids include lysine and arginine; amino acids with uncharged polar head groups having similar hydrophilicity values include the following: leucine, isoleucine, valine; glycine, analine; asparagine, glutamine; serine, threonine; phenylalanine, tyrosine. As used herein, a functionally equivalent Flk-1 refers to a receptor which binds to VEGF or fragments, but not necessarily with the same binding affinity of its counterpart native Flk-1.

The DNA sequences of the invention may be engineered in order to alter the Flk-1 coding sequence for a variety of ends including but not limited to alterations which modify processing and expression of the gene product. For example, mutations may be introduced using techniques which are well. known in the art, e.g. site-directed mutagenesis, to insert new restriction sites, to alter glycosylation patterns, phosphorylation, etc. For example, in certain expression systems such as yeast, host cells may over glycosylate the gene product. When using such expression systems it may be preferable to alter the Flk-1 coding sequence to eliminate any N-linked glycosylation site.

In an alternate embodiment of the invention, the coding sequence of Flk-1 could be synthesized in whole or in part, using chemical methods well known in the art. See, for example, Caruthers, et al., 1980, Nuc. Acids Res. Symp. Ser. 7:215-233; Crea and Horn, 180, Nuc. Acids Res. **9**(10):2331; Matteucci and Caruthers, 1980, Tetrahedron Letters 21:719; and Chow and Kempe, 1981, Nuc. Acids Res. 9(12):2807-2817. Alternatively, the protein itself could be produced using chemical methods to synthesize the Flk-1 amino acid sequence in whole or in part. For example, peptides can be synthesized by solid phase techniques, cleaved from the resin, and purified by preparative high performance liquid chromatography. (E.g., see Creighton, 1983, Proteins Structures And Molecular Principles, W.H. Freeman and Co., N.Y. pp. 50-60). The composition of the synthetic peptides may be confirmed by amino acid analysis or sequencing (e.g., the Edman degradation procedure; see Creighton, 1983, Proteins, Structures and Molecular Principles, W.H. Freeman and Co., N.Y., pp. 34-49.

### 5.2. EXPRESSION OF Flk-1 RECEPTOR AND GENERATION OF CELL LINES THAT EXPRESS Flk-1

In order to express a biologically active Flk-1, the nucleotide sequence coding for Flk-1, or a functional equivalent as described in Section 5.1 supra, is inserted into an appropriate expression vector, i.e., a vector which contains the necessary elements for the transcription and translation of the inserted coding sequence. The Flk-1 gene products as well as host cells or cell lines transfected or transformed with recombinant Flk-1 expression vectors can be used for a variety of purposes. These include but are not limited to generating antibodies (i.e., monoclonal or polyclonal) that bind to the receptor, including those that competitively inhibit binding of VEGF and "neutralize" activity of Flk-1 and the screening and selection of VEGF analogs or drugs that act via the Flk-1 receptor; etc.

### 5.2.1. EXPRESSION SYSTEMS

Methods which are well known to those skilled in the art can be used to construct expression vectors containing the Flk-1 coding sequence and appropriate transcriptional/translational control signals. These methods include in vitro recombinant DNA techniques, synthetic techniques and in vivo recombination/genetic recombination. See, for example, the techniques described in Maniatis et al., 1989, Molecular Cloning A Laboratory Manual, Cold Spring Harbor Laboratory, N.Y. and Ausubel et al., 1989, Current Protocols in Molecular Biology, Greene Publishing Associates and Wiley Interscience, N.Y.

A variety of host-expression vector systems may be utilized to express the Flk-1 coding sequence. These include but are not limited to microorganisms such as bacteria transformed with recombinant bacteriophage DNA, plasmid DNA or cosmid DNA expression vectors containing the Flk-1 coding sequence; yeast transformed with recombinant yeast expression vectors containing the Flk-1 coding sequence; insect cell systems infected with recombinant virus expression vectors (e.g., baculovirus) containing the Flk-1 coding sequence; plant cell systems infected with recombinant virus expression vectors (e.g., cauliflower mosaic virus, CaMV; tobacco mosaic virus, TMV) or transformed with'recombinant plasmid expression vectors (e.g., Ti plasmid) containing the Flk-1 coding sequence; or animal cell systems infected with recombinant virus expression vectors (e.g., adenovirus, vaccinia virus) including cell lines engineered to contain multiple copies of the Flk-1 DNA either stably amplified (CHO/dhfr) or unstably amplified in double-minute chromosomes (e.g., murine cell lines).

The expression elements of these systems vary in their strength and specificities. Depending on the host/vector system utilized, any of a number of suitable transcription and translation elements, including constitutive and inducible promoters, may be used in the expression vector. For example, when cloning in-bacterial systems, inducible promoters such as pL of bacteriophage λ, plac, ptrp, ptac (ptrp-lac hybrid promoter) and the like may be used; when cloning in insect cell systems, promoters such as the baculovirus polyhedrin promoter may be used; when cloning in plant cell systems, promoters derived from the genome of plant cells (e.g., heat shock promoters; the promoter for the small subunit of RUBISCO; the promoter for the chlorophyll a/b binding protein) or from plant viruses (e.g., the 35S RNA promoter of CaMV; the coat protein promoter of TMV) may be used; when cloning in mammalian cell systems, promoters derived from the genome of mammalian cells (e.g., metallothionein promoter) or from mammalian viruses (e.g., the adenovirus late promoter; the vaccinia virus 7.5K promoter) may be used; when generating cell lines that contain multiple copies of the Flk-1 DNA SV40-, BPV- and EBV-based vectors may be used with an appropriate selectable marker.

In bacterial systems a number of expression vectors may be advantageously selected depending upon the use intended for the Flk-1 expressed. For example, when large quantities of Flk-1 are to be produced for the generation of antibodies or to screen peptide libraries, vectors which direct the expression of high levels of fusion protein products that are readily purified may be desirable. Such vectors include but are not limited to the E. coli expression vector pUR278 (Ruther et al., 1983, EMBO J. 2:1791), in which the Flk-1 coding sequence may be ligated into the vector in frame with the lac Z coding region so that a hybrid AS-lac Z protein is produced; pIN vectors (Inouye & Inouye, 1985, Nucleic acids Res. 13:3101-3109; Van Heeke & Schuster, 1989, J. Biol. Chem. 264:5503-5509); and the like. pGEX vectors may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The pGEX vectors are designed to include thrombin or factor Xa protease cleavage sites so that the cloned polypeptide of interest can be released from the GST moiety.

In yeast, a number of vectors containing constitutive or inducible promoters may be used. For a review see, Current Protocols in Molecular Biology, Vol. 2, 1988, Ed. Ausubel et al., Greene Publish. Assoc. & Wiley Interscience, Ch. 13; Grant et al., 1987, Expression and Secretion Vectors for Yeast, in Methods in Enzynology, Eds. Wu & Grossman, 1987, Acad. Press, N.Y., Vol. 153, pp. 516-544; Glover, 1986, DNA Cloning, Vol. II, IRL Press, Wash., D.C., Ch. 3; and Bitter, 1987, Heterologous Gene Expression in Yeast, Methods in Enzymology, Eds. Berger & Kimmel, Acad. Press, N.Y., Vol. 152, pp. 673-684; and The Molecular Biology of the Yeast Saccharomyces, 1982, Eds. Strathern et al., Cold Spring Harbor Press, Vols. I and II.

In cases where plant expression vectors are used, the expression of the Flk-1 coding sequence may be driven by any of a number of promoters. For example, viral promoters such as the 35S RNA and 19S RNA promoters of CaMV (Brisson et al., 1984, Nature 310:511-514), or the coat protein promoter of TMV (Takamatsu et al., 1987, EMBO J. 6:307-311) may be used; alternatively, plant promoters such as the small subunit of RUBISCO (Coruzzi et al., 1984, EMBO J. 3:1671-1680; Broglie et al., 1984, Science 224:838-843); or heat shock promoters, e.g., soybean hsp17.5-E or hsp17.3-B (Gurley et al., 1986, Mol. Cell. Biol. 6:559-565) may be used. These constructs can be introduced into plant cells using Ti plasmids, Ri plasmids, plant virus vectors, direct DNA transformation, microinjection, electroporation, etc. For reviews of such techniques see, for example, Weissbach & Weissbach, 1988, Methods for Plant Molecular Biology, Academic Press, NY, Section VIII, pp. 421-463; and Grierson & Corey, 1988, Plant Molecular Biology, 2d Ed., Blackie, London, Ch. 7-9.

An alternative expression system which could be used to express Flk-1 is an insect system. In one such system, Autographa californica nuclear polyhidrosis virus (AcNPV) is used as a vector to express foreign genes. The virus grows in Spodoptera frugiperda cells. The Flk-1 coding sequence may be cloned into non-essential regions (for example the polyhedrin gene) of the virus and placed under control of an AcNPV promoter (for example the polyhedrin promoter). Successful insertion of the Flk-1 coding sequence will result in inactivation of the polyhedrin gene and production of non-occluded recombinant virus (i.e., virus lacking the proteinaceous coat coded for by the polyhedrin gene). These recombinant viruses are then used to infect Spodoptera frugiperda cells in which the inserted gene is expressed. (E.g., see Smith et al., 1983, J. Viol. 46:584; Smith, U.S. Patent No. 4,215,051).

In mammalian host cells, a number of viral based expression systems may be utilized. In cases where an adenovirus is used as an expression vector, the Flk-1 coding sequence may be ligated to an adenovirus transcription/translation control complex, e.g., the late promoter and tripartite leader sequence. This chimeric gene may then be inserted in the adenovirus genome by in vitro or in vivo recombination. Insertion in a non-essential region of the viral genome (e.g., region E1 or E3) will result in a recombinant virus that is viable and capable of expressing Flk-1 in infected hosts. (E.g., See Logan & Shenk, 1984, Proc. Natl. Acad. Sci. (USA) 81:3655-3659). Alternatively, the vaccinia 7.5K promoter may be used. (See, e.g., Mackett et al., 1982, Proc. Natl. Acad. Sci. (USA) 79:7415-7419; Mackett et al., 1984, J. Virol. 49:857-864; Panicali et al., 1982, Proc. Natl. Acad. Sci. 79:4927-4931).

Specific initiation signals may also be required for efficient translation of inserted Flk-1 coding sequences. These signals include the ATG initiation codon and adjacent sequences. In cases where the entire Flk-1 gene, including its own initiation codon and adjacent sequences, is inserted into the appropriate expression vector, no additional translational control signals may be needed. However, in cases where only a portion of the Flk-1 coding sequence is inserted, exogenous translational control signals, including the ATG initiation codon, must be provided. Furthermore, the initiation codon must be in phase with the reading frame of the Flk-1 coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, Methods in Enzymol. 153:516-544).

In addition, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein. Different host cells have characteristic and specific mechanisms for the post-translational processing and modification of proteins. Appropriate cells lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, WI38, etc.

For long-term, high-yield production of recombinant proteins, stable expression is preferred. For example, cell lines which stably express the Flk-1 may be engineered. Rather than using expression vectors which contain viral origins of replication, host cells can be transformed with the Flk-1 DNA controlled by appropriate expression control elements (e.g., promoter, enhancer, sequences, transcription terminators, polyadenylation sites, etc.), and a selectable marker. Following the introduction of foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the Flk-1 on the cell surface, and which respond to VEGF mediated signal transduction. Such engineered cell lines are particularly useful in screening VEGF analogs.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk⁻, hgprt⁻ or aprt⁻ cells, respectively. Also, antimetabolite resistance can be used as the basis of selection for dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981), Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147) genes. Recently, additional selectable genes have been described, namely trpB, which allows cells to utilize indole in place of tryptophan; hisD, which allows cells to utilize histinol in place of histidine (Hartman & Mulligan, 1988, Proc. Natl. Acad. Sci. USA 85:8047); and ODC (ornithine decarboxylase) which confers resistance to the ornithine decarboxylase inhibitor, 2-(difluoromethyl)-DL-ornithine, DEMO (McConlogue L., 1987, In: Current Communications in Molecular Biology, Cold Spring Harbor Laboratory ed.).

### 5.2.2. IDENTIFICATION OF TRANSFECTANTS OR TRANSFORMANTS THAT EXPRESS THE Flk-1

The host cells which contain the coding sequence and which express the biologically active gene product may be identified by at least four general approaches; (a) DNA-DNA or DNA-RNA hybridization; (b) the presence or absence of "marker" gene functions; (c) assessing the level of transcription as measured by the expression of Flk-1 mRMA transcripts in the host cell; and (d) detection of the gene product as measured by immunoassay or by its biological activity.

In the first approach, the presence of the Flk-1 coding sequence inserted in the expression vector can be detected by DNA-DNA or DNA-RNA hybridization using probes comprising nucleotide sequences that are homologous to the Flk-1 coding sequence, respectively, or portions or derivatives thereof.

In the second approach, the recombinant expression vector/host system can be identified and selected based upon the presence or absence of certain "marker" gene functions (e.g., thymidine kinase activity, resistance to antibiotics, resistance to methotrexate, transformation phenotype, occlusion body formation in baculovirus, etc.). For example, if the Flk-1 coding sequence is inserted within a marker gene sequence of the vector, recombinants containing the Flk-1 coding sequence can be identified by the absence of the marker gene function. Alternatively, a marker gene can be placed in tandem with the Flk-1 sequence under the control of the same or different promoter used to control the expression of the Flk-1 coding sequence. Expression of the marker in response to induction or selection indicates expression of the Flk-1 coding sequence.

In the third approach, transcriptional activity for the Flk-1 coding region can be assessed by hybridization assays. For example, RNA can be isolated and analyzed by Northern blot using a probe homologous to the Flk-1 coding sequence or particular portions thereof. Alternatively, total nucleic acids of the host cell may be extracted and assayed for hybridization to such probes.

In the fourth approach, the expression of the Flk-1 protein product can be assessed immunologically, for example by Western blots, immunoassays such as radioimmuno-precipitation, enzyme-linked immunoassays and the like. The ultimate test of the success of the expression system, however, involves the detection of the biologically active Flk-1 gene product. A number of assays can be used to detect receptor activity including but not limited to VEGF binding assays; and VEGF biological assays using engineered cell lines as the test . substrate.

### 5.3. USES OF THE Flk-1 RECEPTOR AND ENGINEERED CELL LINES

Angiogenesis, the growth of new blood capillary vessels, is required for a number of physiological processes ranging from wound healing, tissue and organ regeneration, placental formation after pregnancy and embryonic development. Abnormal proliferation of blood vessels is an important component of a variety 'of diseases such as rheumatoid arthritis, retinopathies, and psoriasis. Angiogenesis is also an important factor in the growth and metastatic activity of solid tumors that rely on vascularization. Therefore, inhibitors of angiogenesis may be used therapeutically for the treatment of diseases resulting from or accompanied by abnormal growth of blood vessels and for treatments of malignancies involving growth and spread of solid tumors.

### 5.4 USE OF DOMINANT NEGATIVE Flk-1 MUTANTS IN GENE THERAPY

Receptor dimerization induced by ligands, is thought to provide an allosteric regulatory signal that functions to couple ligand binding to stimulation of kinase activity. Defective receptors can function as dominant negative mutations by suppressing the activation and response of normal receptors by formation of unproductive heterodimers. Therefore, defective receptors can be engineered into recombinant viral vectors and used in gene therapy in individuals that inappropriately express Flk-1.

In an embodiment of the invention, mutant forms of the Flk-1 molecule having a dominant negative effect may be identified by expression in selected cells. Deletion or missense mutants of Flk-1 that retain the ability to form dimers with wild type Flk-1 protein but cannot function in signal transduction may be used to inhibit the biological activity of the endogenous wild type Flk-1. For example, the cytoplasmic kinase domain of Flk-1 may be deleted resulting in a truncated Flk-1 molecule that is still able to undergo dimerization with endogenous wild type receptors but unable to transduce a signal.

Abnormal proliferation of blood vessels is an important component of a variety of pathogenic disorders such as rheumatoid arthritis, retinopathies and psoriasis. Uncontrolled angiogenesis is also an important factor in the growth and metastases of solid tumors. Recombinant viruses may be engineered to express dominant negative forms of Flk-1 which may be used to inhibit the activity of the wild type endogenous Flk-1. These viruses may be used therapeutically for treatment of diseases resulting from aberrant expression or activity of Flk-1.

Expression vectors derived from viruses such as retroviruses, vaccinia virus, adeno-associated virus, herpes viruses, or bovine papilloma virus, may be used for delivery of recombinant Flk-1 into the targeted cell population. Methods which are well known to those skilled in the art can be used to construct recombinant viral vectors containing Flk-1 coding sequence. See, for example, the techniques described in Maniatis et al., 1989, Molecular Cloning A Laboratory Manual, Cold Spring Harbor Laboratory, N.Y. and Ausubel et al., 1989, Current Protocols in Molecular-Biology, Greene Publishing Associates and Wiley Interscience, N.Y. Alternatively, recombinant Flk-1 molecules can be reconstituted into liposomes for delivery to target cells.

In a specific embodiment of the invention, a deletion mutant of the Flk-1 receptor was engineered into a recombinant retroviral vector. Two clonal isolates designated pLXSN Flk-1 TM cl.1 and pLXSN Flk-1 TM cl.3 contain a truncated Flk-1 receptor lacking the 561 COOH-terminal amino acids. To obtain virus producing cell lines, PA37 cells were transfected with the recombinant vectors and, subsequently, conditioned media containing virus were used to infect GPE cells.

To test whether expression of signaling-defective mutants inhibits endogenous Flk-1 receptor activity, C6 rat gliobastoma cells (tumor cells) and mouse cells producing the recombinant retroviruses were mixed and injected subcutaneously into nude mice. Normally, injection of tumor cells into nude mice would result in proliferation of the tumor cells and vascularization of the resulting tumor mass. Since Flk-1 is believed to be essential for formation of blood vessels, blocking Flk-1 activity by expression of a truncated receptor, might function to inhibit vascularization of the developing tumor and, thereby, inhibit its growth. As illustrated in Figures 13 and 14, coinjection of virus producing cells, expressing a truncated Flk-1 receptor, significantly inhibits the growth of the tumor as compared to controls receiving only tumor cells.

### 6. EXAMPLE: CLONING AND EXPRESSION PATTERNS OF Flk-1, A HIGH AFFINITY RECEPTOR FOR VEGF

The subsection below describes the cloning and characterization of the Flk-1 cDNA clone. Northern blot and *in situ* hybridization analyses indicate that Flk-1 is expressed in endothelial cells. Cross-linking and ligand binding experiments further indicate that Flk-1 is a high affinity receptor for VEGF.

### 6.1. MATERIALS AND METHODS

### 6.1.1. cDNA CLONING OF Flk-1

DNA extracted from λgt10 cDNA library of day 8.5 mouse embryos (Fahrner et al., 1987, EMBO. J. 6:1497-1508) was used as template for polymerase chain reaction (PCR; Saiki, R.K. et al., 1985 Science 230:1350-1354). In an independent approach cDNA of capillary endothelial cells that had been isolated from the brain of postnatal day 4-8 mice was used for amplification (Risau, W., 1990 In: development of the Vascular system. Issues Biomed. Basel Karger 58-68 and Schnürch et al., unpublished) Degenerated primers were designed on the basis of high amino acid homologies within the kinase domain shared by all RTKs (Wilks, A.F., 1989, Proc. Natl. Acad. Sci. U.S.A. 86:1603-1607).

Full length cDNA clones of Flk-1 were isolated from another day 8.5 mouse embryo cDNA library, which had been prepared according to the method of Okayama and Berg (1983), and a day 11.5 mouse embryo λgt11 library (Clonetech) using the ³²P-labeled (Feinberg, A.P. and Vogelstein, B. 1983 Anal. Biochen. 132:6-13) 210-bp PCR fragment.

### 6.1.2. MOUSE EMBRYOS

Balb/c mice were mated overnight and the morning of vaginal plug detection was defined as 1/2 day of gestation. For Northern blot analysis the frozen embryos were homogenized in 5 M guanidinium thiocyanate and RNA was isolated as described (Ullrich, A. et al., 1985, Nature 313:756-761). For *in situ* hybridization, the embryos were embedded in Tissue-Tek (Miles), frozen on the surface of liquid nitrogen and stored at -70C prior to use.

### 6.1.3. PREPARATION OF PROBES

The 5'-located, 2619 bp of the receptor cDNA were. subcloned in the pGem3Z vector (Promega) as an EcoR1/BamH1 fragment. The probe for Northern blot hybridization was prepared by labelling the cDNA fragment with α-³²PdATP (Amersham) by random hexanucleotide priming (Boehringer; Feinberg, A.P. and Vogelstein, B., 1983 Anal. Biochem. 132:6-13).

For *in situ* hybridization a single-strand antisense DNA probe was prepared as described by Schnürch and Risau (Development, 1991 111:1143-54). The plasmid was linearized at the 3' end of the cDNA and a sense transcript was synthesized using SP6 RNA polymerase (Boehringer). The DNA was degraded using DNAase (RNAase free preparation, Boehringer Mannheim). With the transcript, a random-primed cDNA synthesis with a α-³⁵S dATP (Amersham) was performed by reverse transcription with MMLV reverse transcriptase (BRL). To obtain small cDNA fragments of about 100 bp in average suitable for *in situ* hybridization, a high excess of primer was used. Subsequently the RNA transcript was partially hydrolyzed in 100 mM NaOH for 20 minutes at 70°C, and the probe was neutralized with the same amount of HCl and purified with a Sephadex C50 column. After ethanol precipitation the probe was dissolved at a final specific activity of 5x10⁵ cpm. For control hybridization a sense probe was prepared with the same method.

### 6.1.4. RNA EXTRACTION AND NORTHERN ANALYSIS

Total cytoplasmic RNA was isolated according to the acidic phenol-method of Chromczynski and Sacchi (1987). Poly(A⁺) RNA aliquots were electrophoresed in 1.2% agarose formaldehyde (Sambrook, J. et al., 1989 Molecular Cloning: A Laboratory Manual 2nd ed. Cold Spring Harbor Laboratory Press) gels and transferred to nitrocellulose membranes (Schleicher & Schuell), Hybridizations were performed overnight in 50% formamide, 5 x SSC (750mM sodium chloride, 75mM sodium citrate), 5 x Denhardt's (0.1% Ficoll 400, 0.1% polyvinylpryollidone, 0.1% BSA) and -0.5% SDS at 42°C with 1-3x10⁶ cpm-ml-¹ of ³²P-Random primed DNA probe, followed by high stringency washes in 0.2 x SSC, 0.5% SDS at 52°C. The filters were exposed for 4 to 8 days.

### 6.1.5. IN SITU HYBRIDIZATION

Subcloning postfixation and hybridization was essentially performed according to Hogan et al. (1986). 10 µm thick sections were cut at -18°C on a Leitz cryostat. For prehybridization treatment no incubation with 0.2M HCl for removing the basic proteins was performed. Sections were incubated with the ³⁵S-cDNA probe (5x10⁴cpm/µl) at 52°C in a buffer containing 50% formamide, 300 mM NuCl, 10 mM Tris-HCl, 10 mM NaPO₄ (pH 6.8), 5 mM EDTA, 0.02% Ficoll 400, 0.01% polyvinylprolidone 0.02% BSA 10 m /ml yeast RNA, 10% dextran sulfate, and 10 mM NaCl, 10 mM Tris-HCl, 10 mM NaPO₄ (pH 6.8), 5 mM EDTA, 10 Mm DTT at 52°C). For autoradiography, slides were coated with Kodak NTB2 film emulsion and exposed for eight days. After developing, the sections were counterstained and toluidine blue or May-Grinwald.

### 6.1.6. PREPARATION OF ANTISERA

The 3' primed EcoRV/HindII fragment comprising the 128 C-terminal amino acids of Flk-1 was subcloned in the fusion protein expression vector pGEX3X (Smith, D.B. and Johnson, K.S., 1990 Gene. 67:31-40; Pharmacia). The fusion protein was purified as described and used for immunizing rabbits. After the second boost the rabbits were bled and the antiserum was used for immunoprecipitation.

### 6.1.7. TRANSIENT EXPRESSION OF Flk-1 IN COS-1 CELLS

Transfection of COS-1 cells was performed essentially as described by Chen and Okayama (1987 Mol. Cell. Biol. 7:2745-2752) and Gorman et al. (1989 Virology 171:377-385). Briefly, cells were seeded to a density of 1.0 x 10⁶ per 10-cm dish and incubated overnight in DMEM containing 10% fetal calf serum (Gibco). 20 µg of receptor cDNA cloned into a cytomegalovirus promotor driven expression vector was mixed in 0.5 ml of 0.25 M CaCa₂, 0.5 ml of 2 x BBS (280 mM NaCl, 1.5 mM Na₂HPO₄, 50 mM BES, pH 6.96 and incubated for 30 min at room temperature. The calcium phosphate/DNA solution was then added to the cells, swirled gently, and incubated for 18 hours at 37°C under 3% CO₂. For ligand binding experiments, the cells were removed from the plate and treated as described below.

To obtain VEGF conditioned media, cells were transfected in 15-cm dishes. Media was collected after 48 h and VEGF was partially purified by affinity chromatography using heparin High Trap TM columns (Pharmacia) and concentrated by ultrafiltration (Ferrara, N. and Henzel, W.J. 1989 Biochem. Biophys. Res. Comm. 161:851-858). The concentration of VEGF was determined by a ligand competition assay with bovine aortic endothelial cells.

For autophosphorylation assays, cells were seeded in 6-well dishes (2x10⁵ cells per well), transfected as described above, and starved for 24 h in DMEM containing 0.5% fetal calf serum. The cells were then treated with 500 pM VEGF for 10 min. at 37°C or left untreated and were subsequently lysed as described by Kris et al. (1985). Flk-1 was immunoprecipitated with an antiserum raised in rabbits against the C-terminus of the receptor. The immunoprecipitates were separated on a 7.5% SDS polyacrylamide gel, transferred to nitrocellulose, and incubated with a mouse monoclonal antibody directed . against phosphotyrosine (5E2; Fendly, B.M. et al., 1990 Cancer Research 50:1550-1558). Protein bands were visualized using horseradish peroxidase coupled goat anti-mouse antibody and the ECL™ (Amersham) detection system.

### 6.1.8. RADIOIODINATION OF VEGF

Recombinant human VEGF (5 µg; generously provided by Dr. H. Weich) was dissolved in 110 µl sodium phosphate buffer pH 76, and iodinated by the procedure of Hunter and Greenwood (1962). The reaction products were separated from the labeled protein by passage over a sephadex G50 column, pre-equilibrated with phosphate buffered saline (PBS) containing 0.7% bovine serum albumin (BSA), and aliquots of the collected fractions were counted before and after precipitation with 20% trichloracetic acid. The purity of the iodinated product was estimated to be superior to 90%, as determined by gel electrophoresis, and the specific activity was 77000 cpm/ng. The bioactivity of the iodinated VEGF was confirmed by comparison with the bioactivities of native VEGF using the tissue factor introduction assay described by Clauss, M. et al. (1990 J. Exp. Med. 172:1535-1545).

### 6.1.9. CROSSLINKING OF VEGF TO Flk-1

COS-1 cells transiently expressing Flk-1 and untransfected COS-1 cells were incubated with 200 pM ¹²⁵I-VEGF at 4°C overnight, then washed twice with PBS and exposed to 0.5 mM disuccinimidyl suberate (DSS) in PBS for 1 h at 4°C. The cells were lysed, Flk-1 immunoprecipitated, and analyzed by electrophoresis on a 7% polytarcylamide gel followed by autoradiography.

### 6.1.10. VEGF BINDING

Ligand binding experiments were performed as described previously (Schumacher, R. et al., 1991, J. Biol. Chem. 266:19288-19295), COS-1 cells were grown in a 15-cm culture dish in DMEM for 48h after transfection. Cells were then washed carefully with PBS and incubated with 5 ml of 25 mM EDTA in PBS for 10 min. Cells were then removed from the plate, washed once with binding buffer (DMEM, 25 mM HEPES, pH 7.5, 0.15% gelatin) and resuspended in 5 ml of binding buffer to determine the cell number. In a total volume of 500 µl this cell suspension was incubated for 90 min at 15°C with 10 pM ¹²⁵I-VEGF, and increasing concentration of unlabeled ligand (from 0 to 7 x 10⁻⁹), which was partially purified from conditioned media of COS-1 cells transiently expressing VEGF (164 amino acid form; Breier et al., 1992). After incubation, cells were washed with PBS 0.1% PBS in the cold. Free ligand was removed by repeated centrifugation and resuspension in binding buffer. Finally, the ¹²⁵I radioactivity bound to the cells were determined in a gamma counter (Riastar). Data obtained were analyzed by the method of Munson, P.J. and Rodbard, D. (1980 Anal. Biochem. 107:220-235).

### 6.1.11. RETROVIRAL VECTORS ENCODING TRANSDOMINANT-NEGATIVE MUTANTS OF Flk-1

Recombinant retroviral vectors were constructed that contained the coding region for amino acids 1 through 806 of the Flk-1 receptor (pLX Flk-1 cl.1 and cl.3, Figure 12). A recombinant virus containing a truncated c-fms receptor mutant (pNTK cfms TM cl.7) was used as a control. To obtain virus producing cells mouse GPE cells were infected with amphotrophic virus-containing conditioned media of PA317 cells that had been transfected with recombinant retroviral DNA. C6 gliobastoma tumor cells were implanted into nude mice either alone or coimplanted with virus producing cells. Injected cell numbers for the two sets of experiments are indicated below. Beginning at the time when the first tumors appeared, tumor volumes were measured every 2 to 3 days to obtain a growth curve.

| Experiment No. 1 | | | |
|---|---|---|---|
| Number of Mice | Number of C6 Cells | Virus-Producer Cell Line | Number of Virus-Cells |
| 4 | 5 x 10⁵ | pLXSN Flk-1 TM cl.3 | 1 x 10⁷ |
| 4 | 5 x 10⁵ | None | 0 |
| 4 | 5 x 10⁵ | pNTK cfms TM cl.7 | 5 x 10⁶ |

| Experiment No. 2 | | | |
|---|---|---|---|
| Number of Mice | Number of C6 Cells | Virus-Producer Cell Line | Number of Virus-Cells |
| 4 | 2 x 10⁶ | pLXSN Flk-1 TM cl.1 | 2 x 10⁷ |
| 4 | 2 x 10⁶ | pLXSM Flk-1 TM cl.3 | 2 x 10⁷ |
| 4 | 2 x 10⁶ | None | 0 |
| 4 | 2 x 10⁶ | pNTK cfms TM cl.7 | 2 x 10⁷ |

### 6.2. RESULTS

### 6.2.1. ISOLATION OF Flk-1

To identify RTKs that are expressed during mouse development, PCR assays using two degenerate oligonucleotide primer pools that were designed on the basis of highly conserved sequences within the kinase domain of RTKs were performed (Hanks, S.K. et al. 1988, Science 241:42-52). DNA extracted from a λgt10 cDNA library of day 8.5 mouse embryos (Fahrner, K. et al., 1987, EMBO. J., 6:1497-1508), a stage in mouse development at which many differentiation processes begin was used as the template in the PCR assays. In a parallel approach, with the intention of identifying RTKs that regulate angiogenesis, similar primers were used for the amplification of RTK cDNA sequences from capillary endothelial-cells that had been isolated from the brains of postnatal day 4-8 mice, a time at which brain endothelial cell proliferation is maximal (Robertson, P.L. et al., 1985, Devel. Brain Res. 23:219-223). Both approaches yielded cDNA sequences (FIG. 11, SEQ. ID NO.:) encoding the recently described fetal liver RTK, Flk-1 (Matthews, W. et al., 1991, Proc. Natl. Acad. Sci. U.S.A. 88:9026-9030). Based on amino acid homology, this receptor is a member of the type III subclass of RTKs (Ullrich, A. and Schlessinger, J. 1990, Cell 61:203-212) and is closely related to human *flt*, which also contains seven immunoglobin-like repeats in its extracellular domain in contrast to other RTKs of that subfamily, which contain only five such repeat structures (Matthews, W. et al., 1991, Proc. Natl. Acad Sci. U.S.A. 88:9026-9030). Sequence comparisons of Flk-1 with KDR (Terman, B.I. et al., 1991, Oncogene 6:1677-1683) and TKr-C (Sarzani, R. et al., 1992, Biochem. Biophys. Res. Comm. 186:706-714) suggest that these are the human and rat homologues of Flk-1, respectively (Figure 1).

### 6.2.2 EXPRESSION OF Flk-1 mRNA DURING EMBRYONIC DEVELOPMENT

As a first step towards the elucidation of the biological function of Flk-1, the expression of Flk-1 mRNA was analyzed in mouse embryos at different development stages. Northern blot hybridization experiments indicated abundant expression of a major 5.5 kb mRNA between day 9.5 and day 18.5, with an apparent decline towards the end of gestation (Figure 2A). In postnatal day 4-8 brain capillaries Flk-1 mRNA was found to be highly enriched compared to total brain mRNA (Figure 2B).

*In situ* hybridization experiments were performed to obtain more detailed information about the expression of Flk-1 during different embryonal stages. A single-stranded antisense, 2619-nucleotide-long DNA probe comprising the Flk-1 extracellular domain was used as a probe because it generated the most specific hybridization signals. As an example, a parasagittal section of a day 14.5 embryo is shown in Figure 3. High levels of hybridization were detected in the ventricle of the heart, the lung, and the meninges; other tissues such as brain, liver, and mandible appeared to contain fewer cells expressing Flk-1 mRNA. Thin strands of Flk-1 expression were also observed in the intersegmental regions of the vertebrae and at the inner surface of the atrium and the aorta. Higher magnification revealed that the expression of Flk-1 seemed to be restricted to capillaries and blood vessels. Closer examination of the heart, for example, showed positive signals only in the ventricular capillaries and endothelial lining of the atrium (Figure 4A). In the lung, Flk-1 expression was detected in peribronchial capillaries, but was absent from bronchial epithelium (Figure 4D). The aorta showed strong hybridization in endothelial cells, but not in the muscular layer (Figure 4C).

### 6.2.3. EXPRESSION OF Flk-1 DURING ORGAN ANGIOGENESIS

The neuroectoderm in the telencephalon of a day 11.5 mouse embryo is largely avascular; the first vascular sprouts begin to radially invade the organ originating from the perineural vascular plexus (Bär, J., 1980, Adv. Anat. Embryol. Cell. Biol. 59:1-62; Risau, W. and Lemmon, V. 1988, Dev. Biol. 125:441-450). At this stage, expression of Flk-1 was high in the perineural vascular plexus and in invading vascular sprouts, as shown in Figure 5A. These in situ hybridization analyses indicated that the proliferating endothelial cells of an angiogenic sprout expressed the Flk-1 mRNA. At day 14.5, when the neuroectoderm is already highly vascularized, numerous . radial vessels as well as branching vessels of the intraneural plexus contained large amounts of Flk-1 mRNA (Figure 5B). At postnatal day 4, when sprouting and endothelial cell proliferation is at its highest, strong expression of Flk-1 mRNA was observed in endothelial cells (Figure 5C). Conversely, in the adult brain when angiogenesis has ceased, Flk-1 expression was very low (Figure 5D) and appeared to be restricted mainly to the ehoroid plexus (Figure 6). In the choroid plexus, cells in the inner vascular layer expressed Flk-1 mRNA, while epithelial cells did not (Figure 6A, B).

The embryonic kidney is vascularized by an angiogenic process (Ekblom, P. et al., 1982, Cell Diff. 11:35-39). Glomerular and peritubular capillaries develop synchronously with epithelial morphogenesis. In the postnatal day 4 kidney, in addition to other capillaries, prominent expression of Flk-1 was observed in the presumptive glomerular capillaries (Figure 7A). This expression persisted in the adult kidney (Figure 7C and D) and then seemed to be more confined to the glomerular compared to the early postnatal kidney.

### 6.2.4. Flk-1 EXPRESSION IN ENDOTHELIAL CELL PROGENITORS

To investigate the possible involvement of Flk-1 in the early stages of vascular development, analysis of embryos at different stages during blood island formation were performed. In a sagittal section of the deciduum of a day 8.5 mouse embryo, Flk-1 expression was detected on maternal blood vessels in the deciduum, in the yolk sac and in the trophectoderm. Flk-1 mRNA was also found in the allantois and inside the embryo, mainly located in that part where mesenchyma is found (Figure 8A). At a higher magnification of the maternal deciduum, high levels of Flk-1 mRNA expression were found in the inner lining of blood vessels, which consist of endothelial cells (Figure 8B). In the yolk sac, hybridization signals were confined to the mesodermal layer, in which the hemangioblasts differentiate (Figure 8C). Figure 8D shows a blood island at higher magnification, in which the peripheral angioblasts expressed a high level of Flk-1 mRNA.

### 6.2.5. Flk-1 IS A HIGH AFFINITY RECEPTOR FOR VEGF

Detailed examination of in situ hybridization results and comparison with those for VEGF recently reported by Breier, G. et al. (1992, Development 114:521-532) revealed a remarkable similarity in expression pattern. Furthermore, Flk-1 expression in the glomerular endothelium and VEGF in the surrounding epithelial cells (Breier, G. et al., 1992, Development 114:521-532) raised the possibility of a paracrine relationship between these cells types and suggested therefore a ligand-receptor relationship for VEGF and Flk-1, respectively. In order to test this hypothesis, the full-length Flk-1 cDNA was cloned into the mammalian expression vector pCMV, which contains transcriptional control elements of the human cytomegalovirus (Gorman, C.M. et al., 1989, Virology 171:377-385). For transient expression of the receptor, the Flk-1 expressing plasmid was then transfected into COS-1 fibroblasts.

Specific binding of VEGF to the Flk-1 RTK was demonstrated by crosslinking and competition binding experiments. Purified ¹²⁵I-labeled VEGF was incubated with COS-1 cells transfected with the pCMV-Flk-1 expression vector. Crosslinking with DSS and subsequent analysis of immunoprecipitation, PAGE, and autoradiography revealed an approximately 220 kD band which was not detected in the control experiment with untransfected COS-1 cells and is likely to represent the VEGF/Flk-1 receptor complex (Figure 9A). In addition, VEGF competed with ¹²⁵I-VEGF binding to Flk-1 expressing COS-1 cells (Figure 9B), whereas untransfected COS-1 cells did not bind ¹²⁵I-VEGF. The interaction of VEGF with the receptor on transfected cells was specific, as PDGF-BB did not compete with binding of ¹²⁵I-VEGF. Analysis of the binding data revealed a Kd of about 10⁻¹⁰ M, suggesting that Flk-1 is a high affinity receptor of VEGF. This finding, together with the Flk-1 and VEGF in situ hybridization results strongly suggests that Flk-1 is a physiologically relevantly receptor for VEGF.

An autophosphorylation assay was performed to confirm the biological relevance of VEGF binding to the Flk-1 receptor. COS1 cells which transiently expressed Flk-1 were starved in DMEM containing 0.5% fetal calf serum for 24h, stimulated with 0.5 mM VEGF, and lysed. The receptors were immunoprecipitated with the Flk-1 specific polyclonal antibody CT128, and then analyzed by SOS-PAGE and subsequent immunoblotting using the antiphosphotyrosine antibody 5E2 (Fendly, B.M. et al., 1990, Cancer Research 50:1550-1558). A shown in Figure 10, VEGF stimulation of Flk-1 expressing cells led to a significant induction of tyrosine phosphorylation of the 180 kD Flk-1 receptor.

### 6.2.6. INHIBITION OF TUMOR GROWTH BY TRANSDOMINANT-NEGATIVE INHIBITION OF Flk-1

The Flk-1 receptor is believed to play a major role in vasculogenesis and angiogenesis. Therefore, inhibition of Flk-1 activity may inhibit vasculogenesis of a developing tumor and inhibit its growth. To test this hypothesis, tumor cells (C6 rat glioblastoma) and mouse cells producing a recombinant retrovirus encoding a truncated Flk-1 receptor were mixed and implanted subcutaneously into nude mice. The implanted C6 glioblastoma cells secrete VEGF which will bind to and activate the Flk-1 receptors expressed on the surface of mouse endothelial cells. In the absence of any inhibitors of vasculogenesis, the endothelial cells will proliferate and migrate towards the tumor cells. Alternatively, if at the time of injection, the tumor cells are co-injected with cells producing recombinant retrovirus encoding the dominant-negative Flk-1, the endothelial cells growing towards the implanted tumor cells will become infected with recombinant retrovirus which may result in dominant-negative Flk-1 mutant expression and inhibition of endogenous Flk-1 signaling. Suppression of endothelial cell proliferation and migration will result in failure of the implanted tumor cells to become vascularized which will lead to inhibition of tumor growth. As shown in Figures 12 and 13, tumor growth is significantly inhibited in mice receiving implantations of cells producing truncated Flk-1 indicating that expression of a truncated Flk-1 receptor can act in a dominant-negative manner to inhibit the activity of endogenous wild-type Flk-1.

The present invention is not to be limited in scope by the exemplified embodiments which are intended as illustrations of single aspects of the invention, and any clones, DNA or amino acid sequences which are functionally equivalent are within the scope of the invention. Indeed, various modifications of the invention in addition to those described herein will. become apparent to those skilled in the art from the foregoing description and accompanying drawings. Such modifications are intended to fall within the scope of the appended claims.

It is also to be understood that all base pair sizes given for nucleotides are approximate and are used for purposes of description.

## Claims

1. A recombinant vector comprising a nucleotide sequence that encodes a Flk-1 receptor lacking the C-terminal cytoplasmic kinase domain, in which said Flk-1 receptor has dominant negative activity which inhibits the cellular effects of VEGF binding.

2. The recombinant vector of claim 1, containing a nucleotide sequence encoding amino acids 1 through 806 of FLK-1, but not amino acids 807 through 1367 of Flk-1.

3. A recombinant vector comprising a nucleotide sequence that encodes a Flk-1 receptor having a mutation in the cytoplasmic kinase domain of the Flk-1 receptor, in which said Flk-1 receptor has dominant negative activity which inhibits the cellular effects of VEGF binding.

4. The recombinant vector of claim 1, 2 or 3 in which the vector is a viral vector.

5. The recombinant vector of claim 4 in which the viral vector is a retrovirus vector.

6. An engineered cell line that contains the recombinant vector of claim 4.

7. An engineered cell line that contains the recombinant vector of claim 5.

8. Use of an effective amount of the recombinant vectors of claims 1, 2 or 3, for the preparation of a composition for modulating the cellular effects of VEGF in a mammal.

9. Use according to claim 8, in which the vectors are reconstituted into liposomes.

10. Use of an effective amount of the recombinant vector of claim 4, for the preparation of a composition for modulating the cellular effects of VEGF in a mammal.

11. Use of an effective amount of the recombinant vector of claim 5, for the preparation of a composition for modulating the cellular effects of VEGF in a mammal.

12. Use of an effective amount of a truncated Plk-1 receptor, for the preparation of a composition for modulating the cellular effects of VEGF in a mammal, in which said Flk-1 receptor has dominant negative activity which inhibits the cellular effects of VEGF binding.

13. Use of an effective amount of a truncated Flk-1 receptor having a mutation in the cytoplasmic kinase domain of the Flk-1 receptor, for the preparation of a composition for modulating the cellular effects of VEGF in a mammal, in which said Flk-1 receptor has dominant negative activity which inhibits the cellular effects of VEGF binding.

14. Use according to claim 8 or 9, in which the Flk-1 receptor is reconstituted into liposomes.

## Patentansprüche

1. Ein rekombinanter Vektor, der eine Nukleotidsequenz umfasst, die einen Flk-1 Rezeptor kodiert, dem die C-terminale zytoplasmatische Kinasedomäne fehlt, in welchem besagter Flk-1 Rezeptor dominant-negative Aktivität hat, welche die zellulären Effekte der VEGF-Bindung inhibiert.

2. Der rekombinante Vektor von Anspruch 1, der eine Nukleotidsequenz enthält, die die Aminosäuren 1 bis 806 von Flk-1 kodiert, aber nicht die Aminosäuren 807 bis 1367 von Flk-1.

3. Ein rekombinanter Vektor, der eine Nukleotidsequenz umfasst, die einen Flk-1 Rezeptor kodiert, der eine Mutation in der zytoplasmatischen Kinasedomäne des Flk-1 Rezeptors hat, in welchem besagter Flk-1 Rezeptor dominant-negative Aktivität hat, welche die zellulären Effekte der VEGF-Bindung inhibiert.

4. Der rekombinante Vektor von Anspruch 1, 2 oder 3, in welchem der Vektor ein viraler Vektor ist.

5. Der rekombinante Vektor von Anspruch 4, in welchem der virale Vektor ein Retrovirus-Vektor ist.

6. Eine manipulierte Zelllinie, die den rekombinanten Vektor von Anspruch 4 enthält.

7. Eine manipulierte Zelllinie, die den rekombinanten Vektor von Anspruch 5 enthält.

8. Verwendung einer wirksamen Menge der rekombinanten Vektoren der Ansprüche 1, 2 oder 3 für die Herstellung einer Zusammensetzung zur Modulierung der zellulären Effekte von VEGF in einem Säuger.

9. Verwendung gemäß Anspruch 8, in welcher die Vektoren in Liposomen rekonstituiert sind.

10. Verwendung einer wirksamen Menge des rekombinanten Vektors von Anspruch 4 für die Herstellung einer Zusammensetzung zur Modulierung der zellulären Effekte von VEGF in einem Säuger.

11. Verwendung einer wirksamen Menge des rekombinanten Vektors von Anspruch 5 für die Herstellung einer Zusammensetzung zur Modulierung der zellulären Effekte von VEGF in einem Säuger.

12. Verwendung einer wirksamen Menge eines trunkierten Flk-1 Rezeptors für die Herstellung einer Zusammensetzung zur Modulierung der zellulären Effekte von VEGF in einem Säuger, in welcher besagter Flk-1 Rezeptor dominant-negative Aktivität hat, welche die zellulären Effekte der VEGF-Bindung inhibiert.

13. Verwendung einer wirksamen Menge eines trunkierten Flk-1 Rezeptors, der eine Mutation in der zytoplasmatischen Kinasedomäne des Flk-1 Rezeptors hat, für die Herstellung einer Zusammensetzung zur Modulierung der zellulären Effekte von VEGF in einem Säuger, in welcher besagter Flk-1 Rezeptor dominant-negative Aktivität hat, welche die zellulären Effekte der VEGF-Bindung inhibiert.

14. Verwendung gemäß Anspruch 8 oder 9, in welcher der Flk-1 Rezeptor in Liposomen rekonstituiert ist.

## Revendications

1. Vecteur recombinant comprenant une séquence de nucléotide qui encode un récepteur Flk-1 manquant le domaine kinase cytoplasmique C terminal dans lequel ledit récepteur Flk-1 présente une activité négative dominante qui inhibe les effets cellulaires de la liaison du VEGF (facteur de croissance endothélial vasculaire).

2. Vecteur recombinant de la revendication 1, contenant une séquence de nucléotide codant les acides aminés de 1 à 806 de Flk-1 mais pas les acides aminés de 807 à 1367 de Flk-1.

3. Vecteur recombinant comprenant une séquence de nucléotide qui code un récepteur Flk-1 ayant une mutation dans le domaine kinase cytoplasmique du récepteur Flk-1 dans lequel ledit récepteur Flk-1 présente une activité négative dominante qui inhibe les effets cellulaires de la liaison du VEGF (facteur de croissance endothélial vasculaire).

4. Vecteur recombinant des revendications 1, 2 ou 3 dans lequel le vecteur est un vecteur viral.

5. Vecteur recombinant de la revendication 4 dans lequel le vecteur viral est un vecteur rétroviral.

6. Ligne de cellules modifiées qui contient le vecteur recombinant de la revendication 4.

7. Ligne de cellules modifiées qui contient le vecteur recombinant de la revendication 5.

8. Utilisation d'une quantité efficace des vecteurs recombinants des revendications 1, 2 ou 3 pour la préparation d'une composition pour moduler les effets cellulaires du VEGF chez un mammifère.

9. Utilisation selon la revendication 8 dans laquelle les vecteurs sont reconstitués en liposomes.

10. Utilisation d'une quantité efficace du vecteur recombinant de la revendication 4 pour la préparation d'une composition pour la modulation des effets cellulaires du VEGF chez un mammifère.

11. Utilisation d'une quantité efficace du vecteur recombinant de la revendication 5 pour la préparation d'une composition pour la modulation des effets cellulaires du VEGF chez un mammifère.

12. Utilisation d'une quantité efficace d'un récepteur Flk-1 tronqué pour la préparation d'une composition pour moduler les effets cellulaires du VEGF chez un mammifère dans laquelle ledit récepteur Flk-1 présente une activité négative dominante qui inhibe les effets cellulaires de la liaison du VEGF.

13. Utilisation d'une quantité efficace d'un récepteur Flk-1 tronqué ayant une mutation dans le domaine kinase cytoplasmique du récepteur Flk-1 dans laquelle ledit récepteur Flk-1 présente une activité négative dominante qui inhibe les effets cellulaires de la liaison du VEGF.

14. Utilisation selon la revendication 8 ou 9, dans laquelle le récepteur Flk-1 est reconstitué en liposomes.


### SEQUENCE LISTING

(1) GENERAL INFORMATION:
   (i) APPLICANT: Ullrich, et al
   (ii) TITLE OF INVENTION: FIK-1 IS A RECEPTOR FOR VASCULAR ENDOTHELIAL GROWTH FACTOR
   (iii) NUMBER OF SEQUENCES: 2
   (iv) CORRESPONDENCE ADDRESS:
      (A) ADDRESSEE: Pennie & Edmonds
      (B) STREET: 1155 Avenue of the Americas
      (C) CITY: New York
      (D) STATE: New York
      (E) COUNTRY: U.S.A.
      (F) ZIP: 10036-2711
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: To be assigned
      (B) FILING DATE: 03-MAR-1993
      (C) CLASSIFICATION:
   (viii) ATTORNEY/AGENT INFORMATION:
      (A) NAME: Coruzzi, Laura A.
      (B) REGISTRATION NUMBER: 30,742
      (C) REFERENCE/DOCKET NUMBER: 7683-034-999
   (ix) TELECOMMUNICATION INFORMATION:
      (A) TELEPHONE: (212) 790-9090
      (B) TELEFAX: (212) 869-8864/9741
      (C) TELEX: 66141 PENNIE
(2) INFORMATION FOR SEQ ID NO:1:
   (i) SEQUENCE CHARACTERISTICS:
      (A) LENGTH: 5470 base pairs
      (B) TYPE: nucleic acid
      (C) STRANDEDNESS: unknown
      (D) TOPOLOGY: unknown
   (ii) MOLECULE TYPE: DNA (genomic)
   (ix) FEATURE:
      (A) NAME/KEY: CDS
      (B) LOCATION: 286..4386
   (xi) SEQUENCE DESCRIPTION: SEQ 10 NO:1:
   TATAGGGCGA ATTGGGTACG GGACCCCCCT CGAGGTCGAC GGTATCGATA AGCTTGATAT    60
   CGAATTCGGG CCCAGACTGT GTCCCGCAGC CGGGATAACC TGGCTGACCC GATTCCGCGG   120
   ACACCGCTGA CAGCCGCGGC TGGAGCCAGG GCGCCGGTGC CCCGCGCTCT CCCCGGTCTT   180
   GCGCTGCGGG GGCCATACCG CCTCTGTGAC TTCTTTGCGG GCCAGGGACG GAGAAGGAGT   240
   CTGTGCCTGA GAAACTGGGC TCTGTGCCCA GGCGCGAGGT GCAGG ATG GAG AGC      294
                                                      Met Glu Ser
                                                        1
   AAG GCG CTG CTA GCT GTC GCT CTG TGG TTC TGC GTG GAG ACC CGA GCC   342
   Lys Ala Leu Leu Ala Val Ala Leu Trp Phe Cys Val Glu Thr Arg Ala
           5                  10                  15
   GCC TCT GTG GGT TTG ACT GGC GAT TTT CTC CAT CCC CCC AAG CTC AGC   390
   Ala Ser Val Gly Leu Thr Gly Asp Phe Leu His Pro Pro Lys Leu Ser
    20                  25                  30                  35
   ACA CAG AAA GAC ATA CTG ACA ATT TTG GCA AAT ACA ACC CTT CAG ATT   438
   Thr Gln Lys Asp Ile Leu Thr Ile Leu Ala Asn Thr Thr Leu Gln Ile
                    40                  45                  50
   ACT TGC AGG GGA CAG CGG GAC CTG GAC TGG CTT TGG CCC AAT GCT CAG   486
   Thr Cys Arg Gly Gln Arg Asp Leu Asp Trp Leu Trp Pro Asn Ala Gln
                55                  60                  65
   CGT GAT TCT GAG GAA AGG GTA TTG GTG ACT GAA TGC GGC GGT GGT GAC   534
   Arg Asp Ser Glu Glu Arg Val Leu Val Thr Glu Cys Gly Gly Gly Asp
            70                  75                  80
   AGT ATC TTC TGC AAA ACA CTC ACC ATT CCC AGG GTG GTT GGA AAT GAT   582
   Ser Ile Phe Cys Lys Thr Leu Thr Ile Pro Arg Val Val Gly Asn Asp
        85                  90                  95
   ACT GGA GCC TAC AAG TGC TCG TAC CGG GAC GTC GAC ATA GCC TCC ACT   630
   Thr Gly Ala Tyr Lys Cys Ser Tyr Arg Asp Val Asp Ile Ala Ser Thr
   100                 105                 110                 115
   GTT TAT GTC TAT GTT CGA GAT TAC AGA TCA CCA TTC ATC GCC TCT GTC   678
   Val Tyr Val Tyr Val Arg Asp Tyr Arg Ser Pro Phe Ile Ala Ser Val
                   120                 125                 130
   AGT GAC CAG CAT GGC ATC GTG TAC ATC ACC GAG AAC AAG AAC AAA ACT   726
   Ser Asp Gln His Gly Ile Val Tyr Ile Thr Glu Asn Lys Asn Lys Thr
               135                 140                 145
   GTG GTG ATC CCC TGC CGA GGG TCG ATT TCA AAC CTC AAT GTG TCT CTT   774
   Val Val Ile Pro Cys Arg Gly Ser Ile Ser Asn Leu Asn Val Ser Leu
           150                 155                 160
   TGC GCT AGG TAT CCA GAA AAG AGA TTT GTT CCG GAT GGA AAC AGA ATT   822
   Cys Ala Arg Tyr Pro Glu Lys Arg Phe Val Pro Asp Gly Asn Arg Ile
       165                 170                 175
   TCC TGG GAC AGC GAG ATA GGC TTT ACT CTC CCC AGT TAC ATG ATC AGC   870
   Ser Trp Asp Ser Glu Ile Gly Phe Thr Leu Pro Ser Tyr Met Ile Ser
   180                 185                 190                 195
   TAT GCC GGC ATG GTC TTC TGT GAG GCA AAG ATC AAT GAT GAA ACC TAT   918
   Tyr Ala Gly Met Val Phe Cys Glu Ala Lys Ile Asn Asp Glu Thr Tyr
                   200                 205                 210
   CAG TCT ATC ATG TAC ATA GTT GTG GTT GTA GGA TAT AGG ATT TAT GAT   966
   Gln Ser Ile Met Tyr Ile Val Val Val Val Gly Tyr Arg Ile Tyr Asp
               215                 220                 225
   GTG ATT CTG AGC CCC CCG CAT GAA ATT GAG CTA TCT GCC GGA GAA AAA  1014
   Val Ile Leu Ser Pro Pro His Glu Ile Glu Leu Ser Ala Gly Glu Lys
           230                 235                 240
   CTT GTC TTA AAT TGT ACA GCG AGA ACA GAG CTC AAT GTG GGG CTT GAT  1062
   Leu Val Leu Asn Cys Thr Ala Arg Thr Glu Leu Asn Val Gly Leu Asp
       245                 250                 255
   TTC ACC TGG CAC TCT CCA CCT TCA AAG TCT CAT CAT AAG AAG ATT GTA    1110
   Phe Thr Trp His Ser Pro Pro Ser Lys Ser His His Lys Lys Ile Val
   260                 265                 270                 275
   AAC CGG GAT GTG AAA CCC TTT CCT GGG ACT GTG GCG AAG ATG TTT TTG    1158
   Asn Arg Asp Val Lys Pro Phe Pro Gly Thr Val Ala Lys Met Phe Leu
                   280                 285                 290
   AGC ACC TTG ACA ATA GAA AGT GTG ACC AAG AGT GAC CAA GGG GAA TAC    1206
   Ser Thr Leu Thr Ile Glu Ser Val Thr Lys Ser Asp Gln Gly Glu Tyr
               295                 300                 305
   ACC TGT GTA GCG TCC AGT GGA CGG ATG ATC AAG AGA AAT AGA ACA TTT    1254
   Thr Cys Val Ala Ser Ser Gly Arg Met Ile Lys Arg Asn Arg Thr Phe
           310                 315                 320
   GTC CGA GTT CAC ACA AAG CCT TTT ATT GCT TTC GGT AGT GGG ATG AAA    1302
   Val Arg Val His Thr Lys Pro Phe Ile Ala Phe Gly Ser Gly Met Lys
       325                 330                 335
   TCT TTG GTG GAA GCC ACA GTG GGC AGT CAA GTC CGA ATC CCT GTG AAG    1350
   Ser Leu Val Glu Ala Thr Val Gly Ser Gln Val Arg Ile Pro Val Lys
   340                 345                 350                 355
   TAT CTC AGT TAC CCA GCT CCT GAT ATC AAA TGG TAC AGA AAT GGA AGG    1398
   Tyr Leu Ser Tyr Pro Ala Pro Asp Ile Lys Trp Tyr Arg Asn Gly Arg
                   360                 365                 370
   CCC ATT GAG TCC AAC TAC ACA ATG ATT GTT GGC GAT GAA CTC ACC ATC    1446
   Pro Ile Glu Ser Asn Tyr Thr Met Ile Val Gly Asp Glu Leu Thr Ile
               375                 380                 385
   ATG GAA GTG ACT GAA AGA GAT GCA GGA AAC TAC ACG GTC ATC CTC ACC    1494
   Met Glu Val Thr Glu Arg Asp Ala Gly Asn Tyr Thr Val Ile Leu Thr
           390                 395                 400
   AAC CCC ATT TCA ATG GAG AAA CAG AGC CAC ATG GTC TCT CTG GTT GTG    1542
   Asn Pro Ile Ser Met Glu Lys Gln Ser His Met Val Ser Leu Val Val
       405                 410                 415
   AAT GTC CCA CCC CAG ATC GGT GAG AAA GCC TTG ATC TCG CCT ATG GAT    1590
   Asn Val Pro Pro Gln Ile Gly Glu Lys Ala Leu Ile Ser Pro Met Asp
   420                 425                 430                 435
   TCC TAC CAG TAT GGG ACC ATG CAG ACA TTG ACA TGC ACA GTC TAC GCC    1638
   Ser Tyr Gln Tyr Gly Thr Met Gln Thr Leu Thr Cys Thr Val Tyr Ala
                   440                 445                 450
   AAC CCT CCC CTG CAC CAC ATC CAG TGG TAC TGG CAG CTA GAA GAA GCC    1686
   Asn Pro Pro Leu His His Ile Gln Trp Tyr Trp Gln Leu Glu Glu Ala
               455                 460                 465
   TGC TCC TAC AGA CCC GGC CAA ACA AGC CCG TAT GCT TGT AAA GAA TGG    1734
   Cys Ser Tyr Arg Pro Gly Gln Thr Ser Pro Tyr Ala Cys Lys Glu Trp
           470                 475                 480
   AGA CAC GTG GAG GAT TTC CAG GGG GGA AAC AAG ATC GAA GTC ACC AAA    1782
   Arg His Val Glu Asp Phe Gln Gly Gly Asn Lys Ile Glu Val Thr Lys
       485                 490                 495
   AAC CAA TAT GCC CTG ATT GAA GGA AAA AAC AAA ACT GTA AGT ACG CTG    1830
   Asn Gln Tyr Ala Leu Ile Glu Gly Lys Asn Lys Thr Val Ser Thr Leu
   500                 505                 510                 515
   GTC ATC CAA GCT GCC AAC GTG TCA GCG TTG TAC AAA TGT GAA GCC ATC    1878
   Val Ile Gln Ala Ala Asn Val Ser Ala Leu Tyr Lys Cys Glu Ala Ile
                   520                 525                 530
   AAC AAA GCG GGA CGA GGA GAG AGG GTC ATC TCC TTC CAT GTG ATC AGG     1926
   Asn Lys Ala Gly Arg Gly Glu Arg Val Ile Ser Phe His Val Ile Arg
               535                 540                 545
   GGT CCT GAA ATT ACT GTG CAA CCT GCT GCC CAG CCA ACT GAG CAG GAG     1974
   Gly Pro Glu Ile Thr Val Gln Pro Ala Ala Gln Pro Thr Glu Gln Glu
           550                 555                 560
   AGT GTG TCC CTG TTG TGC ACT GCA GAC AGA AAT ACG TTT GAG AAC CTC     2022
   Ser Val Ser Leu Leu Cys Thr Ala Asp Arg Asn Thr Phe Glu Asn Leu
       565                 570                 575
   ACG TGG TAC AAG CTT GGC TCA CAG GCA ACA TCG GTC CAC ATG GGC GAA     2070
   Thr Trp Tyr Lys Leu Gly Ser Gln Ala Thr Ser Val His Met Gly Glu
   580                 585                 590                 595
   TCA CTC ACA CCA GTT TGC AAG AAC TTG GAT GCT CTT TGG AAA CTG AAT     2118
   Ser Leu Thr Pro Val Cys Lys Asn Leu Asp Ala Leu Trp Lys Leu Asn
                   600                 605                 610
   GGC ACC ATG TTT TCT AAC AGC ACA AAT GAC ATC TTG ATT GTG GCA TTT     2166
   Gly Thr Met Phe Ser Asn Ser Thr Asn Asp Ile Leu Ile Val Ala Phe
               615                 620                 625
   CAG AAT GCC TCT CTG CAG GAC CAA GGC GAC TAT GTT TGC TCT GCT CAA     2214
   Gln Asn Ala Ser Leu Gln Asp Gln Gly Asp Tyr Val Cys Ser Ala Gln
           630                 635                 640
   GAT AAG AAG ACC AAG AAA AGA CAT TGC CTG GTC AAA CAG CTC ATC ATC     2262
   Asp Lys Lys Thr Lys Lys Arg His Cys Leu Val Lys Gln Leu Ile Ile
       645                 650                 655
   CTA GAG CGC ATG GCA CCC ATG ATC ACC GGA AAT CTG GAG AAT CAG ACA     2310
   Leu Glu Arg Met Ala Pro Met Ile Thr Gly Asn Leu Glu Asn Gln Thr
   660                 665                 670                 675
   ACA ACC ATT GGC GAG ACC ATT GAA GTG ACT TGC CCA GCA TCT GGA AAT     2358
   Thr Thr Ile Gly Glu Thr Ile Glu Val Thr Cys Pro Ala Ser Gly Asn
                   680                 685                 690
   CCT ACC CCA CAC ATT ACA TGG TTC AAA GAC AAC GAG ACC CTG GTA GAA     2406
   Pro Thr Pro His Ile Thr Trp Phe Lys Asp Asn Glu Thr Leu Val Glu
               695                 700                 705
   GAT TCA GGC ATT GTA CTG AGA GAT GGG AAC CGG AAC CTG ACT ATC CGC     2454
   Asp Ser Gly Ile Val Leu Arg Asp Gly Asn Arg Asn Leu Thr Ile Arg
           710                 715                 720
   AGG GTG AGG AAG GAG GAT GGA GGC CTC TAC ACC TGC CAG GCC TGC AAT     2502
   Arg Val Arg Lys Glu Asp Gly Gly Leu Tyr Thr Cys Gln Ala Cys Asn
       725                 730                 735
   GTC CTT GGC TGT GCA AGA GCG GAG ACG CTC TTC ATA ATA GAA GGT GCC     2550
   Val Leu Gly Cys Ala Arg Ala Glu Thr Leu Phe Ile Ile Glu Gly Ala
   740                 745                 750                 755
   CAG GAA AAG ACC AAC TTG GAA GTC ATT ATC CTC GTC GGC ACT GCA GTG     2598
   Gln Glu Lys Thr Asn Leu Glu Val Ile Ile Leu Val Gly Thr Ala Val
                   760                 765                 770
   ATT GCC ATG TTC TTC TGG CTC CTT CTT GTC ATT GTC CTA CGG ACC GTT     2646
   Ile Ala Met Phe Phe Trp Leu Leu Leu Val Ile Val Leu Arg Thr Val
               775                 780                 785
   AAG CGG GCC AAT GAA GGG GAA CTG AAG ACA GGC TAC TTG TCT ATT GTC     2694
   Lys Arg Ala Asn Glu Gly Glu Leu Lys Thr Gly Tyr Leu Ser Ile Val
           790                 795                 800
   ATG GAT CCA GAT GAA TTG CCC TTG GAT GAG CGC TGT GAA CGC TTG CCT 2742
   Met Asp Pro Asp Glu Leu Pro Leu Asp Glu Arg Cys Glu Arg Leu Pro
   805 810 815
   TAT GAT GCC AGC AAG TGG GAA TTC CCC AGG GAC CGG CTG AAA CTA GGA 2790
   Tyr Asp Ala Ser Lys Trp Glu Phe Pro Arg Asp Arg Leu Lys Leu Gly
   820 825 830 835
   AAA CCT CTT GGC CGC GGT GCC TTC GGC CAA GTG ATT GAG GCA GAC GCT 2838
   Lys Pro Leu Gly Arg Gly Ala Phe Gly Gln Val Ile Glu Ala Asp Ala
   840 845 850
   TTT GGA ATT GAC AAG ACA GCG ACT TGC AAA ACA GTA GCC GTC AAG ATG 2886
   Phe Gly Ile Asp Lys Thr Ala Thr Cys Lys Thr Val Ala Val Lys Met
   855 860 865
   TTG AAA GAA GGA GCA ACA CAC AGC GAG CAT CGA GCC CTC ATG TCT GAA 2934
   Leu Lys Glu Gly Ala Thr His Ser Glu His Arg Ala Leu Met Ser Glu
   870 875 880
   CTC AAG ATC CTC ATC CAC ATT GGT CAC CAT CTC AAT GTG GTG AAC CTC 2982
   Leu Lys Ile Leu Ile His Ile Gly His His Leu Asn Val Val Asn Leu
   885 890 895
   CTA GGC GCC TGC ACC AAG CCG GGA GGG CCT CTC ATG GTG ATT GTG GAA 3030
   Leu Gly Ala Cys Thr Lys Pro Gly Gly Pro Leu Met Val Ile Val Glu
   900 905 910 915
   TTC TGC AAG TTT GGA AAC CTA TCA ACT TAC TTA CGG GGC AAG AGA AAT 3078
   Phe Cys Lys Phe Gly Asn Leu Ser Thr Tyr Leu Arg Gly Lys Arg Asn
   920 925 930
   GAA TTT GTT CCC TAT AAG AGC AAA GGG GCA CGC TTC CGC CAG GGC AAG 3126
   Glu Phe Val Pro Tyr Lys Ser Lys Gly Ala Arg Phe Arg Gln Gly Lys
   935 940 945
   GAC TAC GTT GGG GAG CTC TCC GTG GAT CTG AAA AGA CGC TTG GAC AGC 3174
   Asp Tyr Val Gly Glu Leu Ser Val Asp Leu Lys Arg Arg Leu Asp Ser
   950 955 960
   ATC ACC AGC AGC CAG AGC TCT GCC AGC TCA GGC TTT GTT GAG GAG AAA 3222
   Ile Thr Ser Ser Gln Ser Ser Ala Ser Ser Gly Phe Val Glu Glu Lys
   965 970 975
   TCG CTC AGT GAT GTA GAG GAA GAA GAA GCT TCT GAA GAA CTG TAC AAG 3270
   Ser Leu Ser Asp Val Glu Glu Glu Glu Ala Ser Glu Glu Leu Tyr Lys
   980 985 990 995
   GAC TTC CTG ACC TTG GAG CAT CTC ATC TGT TAC AGC TTC CAA GTG GCT 3318
   Asp Phe Leu Thr Leu Glu His Leu Ile Cys Tyr Ser Phe Gln Val Ala
   1000 1005 1010
   AAG GGC ATG GAG TTC TTG GCA TCA AGG AAG TGT ATC CAC AGG GAC CTG 3366
   Lys Gly Met Glu Phe Leu Ala Ser Arg Lys Cys Ile His Arg Asp Leu
   1015 1020 1025
   GCA GCA CGA AAC ATT CTC CTA TCG GAG AAG AAT GTG GTT AAG ATC TGT 3414
   Ala Ala Arg Asn Ile Leu Leu Ser Glu Lys Asn Val Val Lys Ile Cys
   1030 1035 1040
   GAC TTC GGC TTG GCC CGG GAC ATT TAT AAA GAC CCG GAT TAT GTC AGA 3462
   Asp Phe Gly Leu Ala Arg Asp Ile Tyr Lys Asp Pro Asp Tyr Val Arg
   1045 1050 1055
   AAA GGA GAT GCC CGA CTC CCT TTG AAG TGG ATG GCC CCG GAA ACC ATT 3510
   Lys Gly Asp Ala Arg Leu Pro Leu Lys Trp Met Ala Pro Glu Thr Ile
   1060 1065 1070 1075
   TTT GAC AGA GTA TAC ACA ATT CAG AGC GAT GTG TGG TCT TTC GGT GTG 3558
   Phe Asp Arg Val Tyr Thr Ile Gln Ser Asp Val Trp Ser Phe Gly Val
   1080 1085 1090
   TTG CTC TGG GAA ATA TTT TCC TTA GGT GCC TCC CCA TAC CCT GGG GTC 3606
   Leu Leu Trp Glu Ile Phe Ser Leu Gly Ala Ser Pro Tyr Pro Gly Val
   1095 1100 1105
   AAG ATT GAT GAA GAA TTT TGT AGG AGA TTG AAA GAA GGA ACT AGA ATG 3654
   Lys Ile Asp Glu Glu Phe Cys Arg Arg Leu Lys Glu Gly Thr Arg Met
   1110 1115 1120
   CGG GCT CCT GAC TAC ACT ACC CCA GAA ATG TAC CAG ACC ATG CTG GAC 3702
   Arg Ala Pro Asp Tyr Thr Thr Pro Glu Met Tyr Gln Thr Met Leu Asp
   1125 1130 1135
   TGC TGG CAT GAG GAC CCC AAC CAG AGA CCC TCG TTT TCA GAG TTG GTG 3750
   Cys Trp His Glu Asp Pro Asn Gln Arg Pro Ser Phe Ser Glu Leu Val
   1140 1145 1150 1155
   GAG CAT TTG GGA AAC CTC CTG CAA GCA AAT GCG CAG CAG GAT GGC AAA 3798
   Glu His Leu Gly Asn Leu Leu Gln Ala Asn Ala Gln Gln Asp Gly Lys
   1160 1165 1170
   GAC TAT ATT GTT CTT CCA ATG TCA GAG ACA CTG AGC ATG GAA GAG GAT 3846
   Asp Tyr Ile Val Leu Pro Met Ser Glu Thr Leu Ser Met Glu Glu Asp
   1175 1180 1185
   TCT GGA CTC TCC CTG CCT ACC TCA CCT GTT TCC TGT ATG GAG GAA GAG 3894
   Ser Gly Leu Ser Leu Pro Thr Ser Pro Val Ser Cys Met Glu Glu Glu
   1190 1195 1200
   GAA GTG TGC GAC CCC AAA TTC CAT TAT GAC AAC ACA GCA GGA ATC AGT 3942
   Glu Val Cys Asp Pro Lys Phe His Tyr Asp Asn Thr Ala Gly Ile Ser
   1205 1210 1215
   CAT TAT CTC CAG AAC AGT AAG CGA AAG AGC CGG CCA GTG AGT GTA AAA 3990
   His Tyr Leu Gln Asn Ser Lys Arg Lys Ser Arg Pro Val Ser Val Lys
   1220 1225 1230 1235
   ACA TTT GAA GAT ATC CCA TTG GAG GAA CCA GAA GTA AAA GTG ATC CCA 4038
   Thr Phe Glu Asp Ile Pro Leu Glu Glu Pro Glu Val Lys Val Ile Pro
   1240 1245 1250
   GAT GAC AGC CAG ACA GAC AGT GGG ATG GTC CTT GCA TCA GAA GAG CTG 4086
   Asp Asp Ser Gln Thr Asp Ser Gly Met Val Leu Ala Ser Glu Glu Leu
   1255 1260 1265
   AAA ACT CTG GAA GAC AGG AAC AAA TTA TCT CCA TCT TTT GGT GGA ATG 4134
   Lys Thr Leu Glu Asp Arg Asn Lys Leu Ser Pro Ser Phe Gly Gly Met
   1270 1275 1280
   ATG CCC AGT AAA AGC AGG GAG TCT GTG GCC TCG GAA GGC TCC AAC CAG 4182
   Met Pro Ser Lys Ser Arg Glu Ser Val Ala Ser Glu Gly Ser Asn Gln
   1285 1290 1295
   ACC AGT GGC TAC CAG TCT GGG TAT CAC TCA GAT GAC ACA GAC ACC ACC 4230
   Thr Ser Gly Tyr Gln Ser Gly Tyr His Ser Asp Asp Thr Asp Thr Thr
   1300 1305 1310 1315
   GTG TAC TCC AGC GAC GAG GCA GGA CTT TTA AAG ATG GTG GAT GCT GCA 4278
   Val Tyr Ser Ser Asp Glu Ala Gly Leu Leu Lys Met Val Asp Ala Ala
   1320 1325 1330
   GTT CAC GCT GAC TCA GGG ACC ACA CTG CAG CTC ACC TCC TGT TTA AAT 4326
   Val His Ala Asp Ser Gly Thr Thr Leu Gln Leu Thr Ser Cys Leu Asn
   1335 1340 1345
   GGA AGT GGT CCT GTC CCG GCT CCG CCC CCA ACT CCT GGA AAT CAC GAG      4374
   Gly Ser Gly Pro Val Pro Ala Pro Pro Pro Thr Pro Gly Asn His Glu
           1350                1355                1360
   AGA GGT GCT GCT TAGATTTTCA AGTGTTGTTC TTTCCACCAC CCGGAAGTAG          4426
   Arg Gly Ala Ala
       1365
   CCACATTTGA TTTTCATTTT TGGAGGAGGG ACCTCAGACT GCAAGGAGCT TGTCCTCAGG   4486
   GCATTTCCAG AGAAGATGCC CATGACCCAA GAATGTGTTG ACTCTACTCT CTTTTCCATT   4546
   CATTTAAAAG TCCTATATAA TGTGCCCTGC TGTGGTCTCA CTACCAGTTA AAGCAAAAGA   4606
   CTTTCAAACA CGTGGACTCT GTCCTCCAAG AAGTGGCAAC GGCACCTCTG TGAAACTGGA   4666
   TCGAATGGGC AATGCTTTGT GTGTTGAGGA TGGGTGAGAT GTCCCAGGGC CGAGTCTGTC   4726
   TACCTTGGAG GCTTTGTGGA GGATGCGGGC TATGAGCCAA GTGTTAAGTG TGGGATGTGG   4786
   ACTGGGAGGA AGGAAGGCGC AAGTCGCTCG GAGAGCGGTT GGAGCCTGCA GATGCATTGT   4846
   GCTGGCTCTG GTGGAGGTGG GCTTGTGGCC TGTCAGGAAA CGCAAAGGCG GCCGGCAGGG   4906
   TTTGGTTTTG GAAGGTTTGC GTGCTCTTCA CAGTCGGGTT ACAGGCGAGT TCCCTGTGGC   4966
   GTTTCCTACT CCTAATGAGA GTTCCTTCCG GACTCTTACG TGTCTCCTGG CCTGGCCCCA   5026
   GGAAGGAAAT GATGCAGCTT GCTCCTTCCT CATCTCTCAG GCTGTGCCTT AATTCAGAAC   5086
   ACCAAAAGAG AGGAACGTCG GCAGAGGCTC CTGACGGGGC CGAAGAATTG TGAGAACAGA   5146
   ACAGAAACTC AGGGTTTCTG CTGGGTGGAG ACCCACGTGG CGCCCTGGTG GCAGGTCTGA   5206
   GGGTTCTCTG TCAAGTGGCG GTAAAGGCTC AGGCTGGTGT TCTTCCTCTA TCTCCACTCC   5266
   TGTCAGGCCC CCAAGTCCTC AGTATTTTAG CTTTGTGGCT TCCTGATGGC AGAAAAATCT   5326
   TAATTGGTTG GTTTGCTCTC CAGATAATCA CTAGCCAGAT TTCGAAATTA CTTTTTAGCC   5386
   GAGGTTATGA TAACATCTAC TGTATCCTTT AGAATTTTAA CCTATAAAAC TATGTCTACT   5446
   GGTTTCTGCC TGTGTGCTTA TGTT                                          5470
(2) INFORMATION FOR SEQ ID NO:2:
   (i) SEQUENCE CHARACTERISTICS:
      (A) LENGTH: 1367 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Glu Ser Lys Ala Leu Leu Ala Val Ala Leu Trp Phe Cys Val Glu
   1 5 10 15
   Thr Arg Ala Ala Ser Val Gly Leu Thr Gly Asp Phe Leu His Pro Pro
   20 25 30
   Lys Leu Ser Thr Gln Lys Asp Ile Leu Thr Ile Leu Ala Asn Thr Thr
   35 40 45
   Leu Gln Ile Thr Cys Arg Gly Gln Arg Asp Leu Asp Trp Leu Trp Pro
   50 55 60
   Asn Ala Gln Arg Asp Ser Glu Glu Arg Val Leu Val Thr Glu Cys Gly
   65 70 75 80
   Gly Gly Asp Ser Ile Phe Cys Lys Thr Leu Thr Ile Pro Arg Val Val
   85 90 95
   Gly Asn Asp Thr Gly Ala Tyr Lys Cys Ser Tyr Arg Asp Val Asp Ile
   100 105 110
   Ala Ser Thr Val Tyr Val Tyr Val Arg Asp Tyr Arg Ser Pro Phe Ile
   115 120 125
   Ala Ser Val Ser Asp Gln His Gly Ile Val Tyr Ile Thr Glu Asn Lys
   130 135 140
   Asn Lys Thr Val Val Ile Pro Cys Arg Gly Ser Ile Ser Asn Leu Asn
   145 150 155 160
   Val Ser Leu Cys Ala Arg Tyr Pro Glu Lys Arg Phe Val Pro Asp Gly
   165 170 175
   Asn Arg Ile Ser Trp Asp Ser Glu Ile Gly Phe Thr Leu Pro Ser Tyr
   180 185 190
   Met Ile Ser Tyr Ala Gly Met Val Phe Cys Glu Ala Lys Ile Asn Asp
   195 200 205
   Glu Thr Tyr Gln Ser Ile Met Tyr Ile Val Val Val Val Gly Tyr Arg
   210 215 220
   Ile Tyr Asp Val Ile Leu Ser Pro Pro His Glu Ile Glu Leu Ser Ala
   225 230 235 240
   Gly Glu Lys Leu Val Leu Asn Cys Thr Ala Arg Thr Glu Leu Asn Val
   245 250 255
   Gly Leu Asp Phe Thr Trp His Ser Pro Pro Ser Lys Ser His His Lys
   260 265 270
   Lys Ile Val Asn Arg Asp Val Lys Pro Phe Pro Gly Thr Val Ala Lys
   275 280 285
   Met Phe Leu Ser Thr Leu Thr Ile Glu Ser Val Thr Lys Ser Asp Gln
   290 295 300
   Gly Glu Tyr Thr Cys Val Ala Ser Ser Gly Arg Met Ile Lys Arg Asn
   305 310 315 320
   Arg Thr Phe Val Arg Val His Thr Lys Pro Phe Ile Ala Phe Gly Ser
   325 330 335
   Gly Met Lys Ser Leu Val Glu Ala Thr Val Gly Ser Gln Val Arg Ile
   340 345 350
   Pro Val Lys Tyr Leu Ser Tyr Pro Ala Pro Asp Ile Lys Trp Tyr Arg
   355 360 365
   Asn Gly Arg Pro Ile Glu Ser Asn Tyr Thr Met Ile Val Gly Asp Glu
   370 375 380
   Leu Thr Ile Met Glu Val Thr Glu Arg Asp Ala Gly Asn Tyr Thr Val
   385 390 395 400
   Ile Leu Thr Asn Pro Ile Ser Met Glu Lys Gln Ser His Met Val Ser
   405 410 415
   Leu Val Val Asn Val Pro Pro Gln Ile Gly Glu Lys Ala Leu Ile Ser
   420 425 430
   Pro Met Asp Ser Tyr Gln Tyr Gly Thr Met Gln Thr Leu Thr Cys Thr
   435 440 445
   Val Tyr Ala Asn Pro Pro Leu His His Ile Gln Trp Tyr Trp Gln Leu
   450 455 460
   Glu Glu Ala Cys Ser Tyr Arg Pro Gly Gln Thr Ser Pro Tyr Ala Cys
   465 470 475 480
   Lys Glu Trp Arg His Val Glu Asp Phe Gln Gly Gly Asn Lys Ile Glu
   485 490 495
   Val Thr Lys Asn Gln Tyr Ala Leu Ile Glu Gly Lys Asn Lys Thr Val
   500 505 510
   Ser Thr Leu Val Ile Gln Ala Ala Asn Val Ser Ala Leu Tyr Lys Cys
   515 520 525
   Glu Ala Ile Asn Lys Ala Gly Arg Gly Glu Arg Val Ile Ser Phe His
   530 535 540
   Val Ile Arg Gly Pro Glu Ile Thr Val Gln Pro Ala Ala Gln Pro Thr
   545 550 555 560
   Glu Gln Glu Ser Val Ser Leu Leu Cys Thr Ala Asp Arg Asn Thr Phe
   565 570 575
   Glu Asn Leu Thr Trp Tyr Lys Leu Gly Ser Gln Ala Thr Ser Val His
   580 585 590
   Met Gly Glu Ser Leu Thr Pro Val Cys Lys Asn Leu Asp Ala Leu Trp
   595 600 605
   Lys Leu Asn Gly Thr Met Phe Ser Asn Ser Thr Asn Asp Ile Leu Ile
   610 615 620
   Val Ala Phe Gln Asn Ala Ser Leu Gln Asp Gln Gly Asp Tyr Val Cys
   625 630 635 640
   Ser Ala Gln Asp Lys Lys Thr Lys Lys Arg His Cys Leu Val Lys Gln
   645 650 655
   Leu Ile Ile Leu Glu Arg Met Ala Pro Met Ile Thr Gly Asn Leu Glu
   660 665 670
   Asn Gln Thr Thr Thr Ile Gly Glu Thr Ile Glu Val Thr Cys Pro Ala
   675 680 685
   Ser Gly Asn Pro Thr Pro His Ile Thr Trp Phe Lys Asp Asn Glu Thr
   690 695 700
   Leu Val Glu Asp Ser Gly Ile Val Leu Arg Asp Gly Asn Arg Asn Leu
   705 710 715 720
   Thr Ile Arg Arg Val Arg Lys Glu Asp Gly Gly Leu Tyr Thr Cys Gln
   725 730 735
   Ala Cys Asn Val Leu Gly Cys Ala Arg Ala Glu Thr Leu Phe Ile Ile
   740 745 750
   Glu Gly Ala Gln Glu Lys Thr Asn Leu Glu Val Ile Ile Leu Val Gly
   755 760 765
   Thr Ala Val Ile Ala Met Phe Phe Trp Leu Leu Leu Val Ile Val Leu
   770 775 780
   Arg Thr Val Lys Arg Ala Asn Glu Gly Glu Leu Lys Thr Gly Tyr Leu
   785 790 795 800
   Ser Ile Val Met Asp Pro Asp Glu Leu Pro Leu Asp Glu Arg Cys Glu
   805 810 815
   Arg Leu Pro Tyr Asp Ala Ser Lys Trp Glu Phe Pro Arg Asp Arg Leu
   820 825 830
   Lys Leu Gly Lys Pro Leu Gly Arg Gly Ala Phe Gly Gln Val Ile Glu
   835 840 845
   Ala Asp Ala Phe Gly Ile Asp Lys Thr Ala Thr Cys Lys Thr Val Ala
   850 855 860
   Val Lys Met Leu Lys Glu Gly Ala Thr His Ser Glu His Arg Ala Leu
   865 870 875 880
   Met Ser Glu Leu Lys Ile Leu Ile His Ile Gly His His Leu Asn Val
   885 890 895
   Val Asn Leu Leu Gly Ala Cys Thr Lys Pro Gly Gly Pro Leu Met Val
   900 905 910
   Ile Val Glu Phe Cys Lys Phe Gly Asn Leu Ser Thr Tyr Leu Arg Gly
   915 920 925
   Lys Arg Asn Glu Phe Val Pro Tyr Lys Ser Lys Gly Ala Arg Phe Arg
   930 935 940
   Gln Gly Lys Asp Tyr Val Gly Glu Leu Ser Val Asp Leu Lys Arg Arg
   945 950 955 960
   Leu Asp Ser Ile Thr Ser Ser Gln Ser Ser Ala Ser Ser Gly Phe Val
   965 970 975
   Glu Glu Lys Ser Leu Ser Asp Val Glu Glu Glu Glu Ala Ser Glu Glu
   980 985 990
   Leu Tyr Lys Asp Phe Leu Thr Leu Glu His Leu Ile Cys Tyr Ser Phe
   995 1000 1005
   Gln Val Ala Lys Gly Met Glu Phe Leu Ala Ser Arg Lys Cys Ile His
   1010 1015 1020
   Arg Asp Leu Ala Ala Arg Asn Ile Leu Leu Ser Glu Lys Asn Val Val
   1025 1030 1035 1040
   Lys Ile Cys Asp Phe Gly Leu Ala Arg Asp Ile Tyr Lys Asp Pro Asp
   1045 1050 1055
   Tyr Val Arg Lys Gly Asp Ala Arg Leu Pro Leu Lys Trp Met Ala Pro
   1060 1065 1070
   Glu Thr Ile Phe Asp Arg Val Tyr Thr Ile Gln Ser Asp Val Trp Ser
   1075 1080 1085
   Phe Gly Val Leu Leu Trp Glu Ile Phe Ser Leu Gly Ala Ser Pro Tyr
   1090 1095 1100
   Pro Gly Val Lys Ile Asp Glu Glu Phe Cys Arg Arg Leu Lys Glu Gly
   1105 1110 1115 1120
   Thr Arg Met Arg Ala Pro Asp Tyr Thr Thr Pro Glu Met Tyr Gln Thr
   1125 1130 1135
   Met Leu Asp Cys Trp His Glu Asp Pro Asn Gln Arg Pro Ser Phe Ser
   1140 1145 1150
   Glu Leu Val Glu His Leu Gly Asn Leu Leu Gln Ala Asn Ala Gln Gln
   1155 1160 1165
   Asp Gly Lys Asp Tyr Ile Val Leu Pro Met Ser Glu Thr Leu Ser Met
   1170 1175 1180
   Glu Glu Asp Ser Gly Leu Ser Leu Pro Thr Ser Pro Val Ser Cys Met
   1185 1190 1195 1200
   Glu Glu Glu Glu Val Cys Asp Pro Lys Phe His Tyr Asp Asn Thr Ala
   1205 1210 1215
   Gly Ile Ser His Tyr Leu Gln Asn Ser Lys Arg Lys Ser Arg Pro Val
   1220 1225 1230
   Ser Val Lys Thr Phe Glu Asp Ile Pro Leu Glu Glu Pro Glu Val Lys
   1235 1240 1245
   Val Ile Pro Asp Asp Ser Gln Thr Asp Ser Gly Met Val Leu Ala Ser
   1250 1255 1260
   Glu Glu Leu Lys Thr Leu Glu Asp Arg Asn Lys Leu Ser Pro Ser Phe
   1265 1270 1275 1280
   Gly Gly Met Met Pro Ser Lys Ser Arg Glu Ser Val Ala Ser Glu Gly
   1285 1290 1295
   Ser Asn Gln Thr Ser Gly Tyr Gln Ser Gly Tyr His Ser Asp Asp Thr
   1300 1305 1310
   Asp Thr Thr Val Tyr Ser Ser Asp Glu Ala Gly Leu Leu Lys Met Val
   1315 1320 1325
   Asp Ala Ala Val His Ala Asp Ser Gly Thr Thr Leu Gln Leu Thr Ser
   1330 1335 1340
   Cys Leu Asn Gly Ser Gly Pro Val Pro Ala Pro Pro Pro Thr Pro Gly
   1345 1350 1355 1360
   Asn His Glu Arg Gly Ala Ala
   1365